(19) Europäisches Patentamt
European Patent Office
Office européen des brevets

(11) Publication number: **0 201 999**
**A1**

(12) # EUROPEAN PATENT APPLICATION

(21) Application number: 86301986.5

(22) Date of filing: 18.03.86

(51) Int. Cl.[4]: C 07 C 131/00
C 07 C 153/057, C 07 D 333/20
C 07 D 333/24, C 07 D 261/14
C 07 D 249/08, C 07 D 317/32
C 07 D 277/46, C 07 D 285/12
C 07 D 231/12, C 07 D 307/04

**A request for correction has been filed pursuant to Rule 88 EPC. A decision on the request will be taken during the proceedings before the Examing Division.**

(30) Priority: 16.04.85 GB 8509733
28.08.85 GB 8521390
19.12.85 GB 8531283

(43) Date of publication of application:
20.11.86 Bulletin 86/47

(84) Designated Contracting States:
AT BE CH DE FR GB IT LI LU NL SE

(71) Applicant: IMPERIAL CHEMICAL INDUSTRIES PLC
Imperial Chemical House Millbank
London SW1P 3JF(GB)

(72) Inventor: Kay, Ian Trevor
Portelet Kiln Ride Extension
Wokingham Berkshire(GB)

(72) Inventor: Crowley, Patrick Jelf
56 Ellis Road
Crowthorne Berkshire(GB)

(72) Inventor: Bartholomew, David
10 Rances Lane
Wokingham Berkshire(GB)

(72) Inventor: Shephard, Margaret Claire
14 Lambourne Drive
Maidenhead Berkshire(GB)

(72) Inventor: Heaney, Stephen Paul
11 Lillybrooke Crescent
Maidenhead Berkshire(GB)

(74) Representative: Alner, Henry Giveen Hamilton et al,
Imperial Chemical Industries PLC Legal Department: Patents P.O. Box 6 Bessemer Road
Welwyn Garden City Herts, AL7 1HD(GB)

(54) **Substituted 2-cyano-2-oximino-acetamides, processes for their production and their use as fungicides.**

(57) Compounds having the general formula:

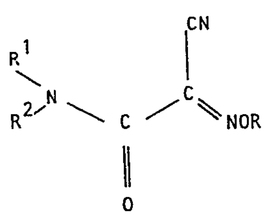

wherein R is H, alkyl, cycloalkyl or cycloalkylalkyl, optionally substituted alkenyl or optionally substituted alkynyl, optionally substituted benzyl, or a metal ion; $R^1$ is an alkenyl, alkynyl, cyanoalkyl, heterocyclyl or allenylmethyl group, any of which groups can bear substituents or $R^1$ is alkyl substituted by alkoxy, halogen, alkylthio, alkoxycarbonyl, carbamoyl, alkylcarbamoyl or thiocarbamoyl; or $R^1$ is alkyl substituted by thiocarbamoyl and additionally by either optionally substituted aryl or optionally substituted heterocyclyl; $R^2$ is H or is a cyanoalkyl, alkenyl, alkynyl or allenylmethyl group, any of which groups can bear substituents; have fungicidal activity.

EP 0 201 999 A1

0201999

TITLE MODIFIED
see front page

HETEROCYCLIC COMPOUNDS

This invention relates to oxime derivatives useful as fungicides, to processes for preparing them, to fungicidal compositions containing them, and to methods of combating fungi, especially fungal infections in plants.

The invention provides compounds having the general formula (I) :

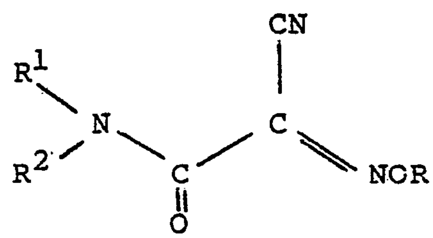

(I)

wherein R is H, alkyl, cycloalkyl or cycloalkylalkyl, optionally substituted alkenyl or optionally substituted alkynyl, optionally substituted benzyl, or a metal ion; $R^1$ is an alkenyl, alkynyl, cyanoalkyl, heterocyclyl or allenylmethyl group, any of which groups can bear substituents or $R^1$ is alkyl substituted by alkoxy, halogen, alkylthio, alkoxycarbonyl, carbamoyl, alkylcarbamoyl or thiocarbamoyl; or $R^1$ is alkyl substituted by thiocarbamoyl and additionally by either optionally substituted aryl or optionally substituted heterocyclyl; $R^2$ is H or is a cyanoalkyl, alkenyl, alkynyl or allenylmethyl group, any of which groups can bear substituents.

The invention in another aspect provides compounds having the general formula (I) :

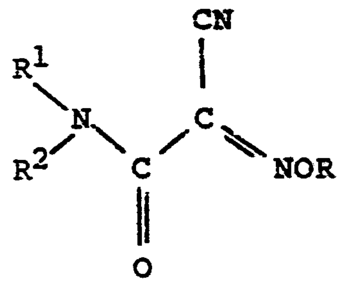

(I)

wherein R is H, alkyl, alkenyl, or alkynyl; $R^1$ is alkenyl, alkynyl, alkoxyalkyl, haloalkyl, alkylthioalkyl, cyanoalkyl, alkoxycarbonylalkyl, alkylcarbamoylalkyl, heterocyclyl, benzyl or allenylmethyl; and $R^2$ is H, alkenyl, alkynyl or allenylmethyl.

In a further aspect the invention provides compounds having the general formula (I) :

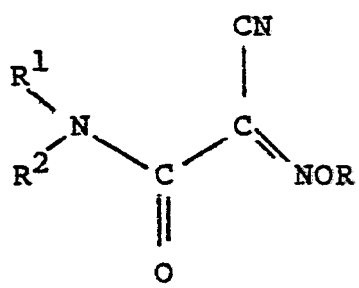

(I)

wherein R is H, alkyl, cycloalkyl or cycloalkylalkyl, optionally substituted alkenyl or alkynyl, optionally substituted benzyl, or a metal ion; $R^1$ is an optionally substituted alkenyl, alkynyl, cyanoalkyl, heterocyclyl or allenylmethyl group or is alkyl substituted by alkoxy, halogen, alkylthio, alkoxycarbonyl, carbamoyl, alkylcarbamoyl or thiocarbamoyl; $R^2$ is H or is an optionally substituted cyanoalkyl, alkenyl, alkynyl or allenylmethyl group.

When R is an alkyl group, it may contain from 1-12 and preferably from 1-6 carbon atoms. Examples of alkyl groups are methyl, ethyl, propyl (n- or iso-propyl) and butyl (n-, sec-, iso- or t-butyl). A particularly

preferred value for R is methyl.

When R is a benzyl group, the benzyl group can be unsubstituted or substituted by halogen (eg. fluorine, chlorine or bromine), $C_{1-4}$ alkyl (eg. methyl, ethyl, iso-propyl or t-butyl), $C_{1-4}$ alkoxy (eg. methoxy or ethoxy), nitro or halo $C_{1-4}$ alkyl (eg. trifluoromethyl). Examples of these groups are benzyl, 4-chlorobenzyl, 2,4-dichlorobenzyl, 2-nitrobenzyl, and 3-trifluoromethylbenzyl. When R is a cycloalkyl group, it may be, for example, a $C_{3-6}$ cycloalkyl group, for example a cyclopropyl, cyclobutyl, cyclopentyl or cyclohexyl group.

When R is cycloalkylalkyl group, it may be, for example, a $C_{3-6}$ cycloalkyl $C_{1-4}$ alkyl group, such as, for example, a cyclopropylmethyl, cyclopentylmethyl or cyclohexylmethyl group.

When R is an optionally substituted alkenyl or alkynyl group, it may be, for example, a $C_{3-5}$ alkenyl or $C_{3-5}$ alkynyl group. Examples include allyl and propargyl. Examples of substituents for a substituted alkenyl or alkynyl group include $C_{1-4}$ alkyl (eg. methyl) or halogen (eg. chlorine, fluorine, bromine or iodine).

When R is a metal ion it may be, for example, sodium, potassium, copper or iron.

When $R^1$ is an optionally substituted alkenyl or alkynyl group, the alkenyl or alkynyl group may contain from 3 to 10 carbon atoms, but preferably contains from 3 to 6 carbon atoms, with allyl and propargyl being particularly preferred groups. Examples of substituents are one or more $C_{1-4}$ alkyl (eg. methyl), halogen (eg. chlorine, fluorine, bromine and iodine) and $C_{1-4}$ alkoxy (eg. methoxy).

When $R^1$ is alkyl substituted by alkoxy, halogen, $C_{1-4}$ alkylthio, $C_{1-4}$ alkoxycarbonyl, carbamoyl, $C_{1-4}$ alkylcarbamoyl or thiocarbamoyl, the alkyl moiety may be a straight or branched chain group having from 1 to 10 carbon

atoms, but preferably having from 1 to 2 carbon atoms, and especially one carbon atom. The alkyl moiety in $R^1$ may be substituted by one or more of any of a combination of the aforementioned groups. Examples of alkoxy substituents are $C_{1-4}$ alkoxy (eg. methoxy or ethoxy) and also include cyclic alkoxy groups such as tetrahydrofuryl, tetrahydropyranyl or 1,3-dioxolanyl where one or more oxygen atoms are contained in a five- or six- membered ring. Examples of halogen substituents are fluorine, chlorine, bromine or iodine. Examples of alkylthio substituents are $C_{1-4}$ alkylthio (eg. methylthio). Examples of alkoxycarbonyl substituents are $C_{1-5}$ alkoxycarbonyl (eg. methoxycarbonyl). Examples of alkylcarbamoyl substituents are $C_{1-5}$ alkylcarbamoyl (eg. methylcarbamoyl).

When $R^1$ is alkyl substituted by thiocarbamoyl, the alkyl group may additionally be substituted by (i) aryl (eg. phenyl) optionally substituted by $C_{1-4}$ alkyl (eg. methyl), halogen (eg. chlorine, fluorine, bromine or iodine), $C_{1-4}$ alkoxy (eg. methoxy), nitro, halo $C_{1-4}$ alkyl (eg. trifluoromethyl), or cyano, or (ii) the alkyl group may additionally be substituted by heterocyclyl (eg. 2-furyl, 2-thienyl) optionally substituted by $C_{1-4}$ alkyl (eg. methyl), halogen (eg. chlorine or bromine), nitro, halo $C_{1-4}$ alkyl (eg. trifluoromethyl) or cyano.

When $R^1$ is optionally substituted cyanoalkyl, the alkyl moiety may contain from 1 to 10 carbon atoms, but preferably contains from 1 to 2 carbon atoms, and especially one carbon atom.

When $R^1$ is cyanoalkyl the alkyl moiety thereof may be substituted by (i) $C_{1-4}$ alkyl (eg. methyl); or (ii) $C_{1-4}$ alkoxy (eg. methoxy or ethoxy), $C_{3-5}$ alkenyloxy (eg. allyloxy), or $C_{3-5}$ alkynyloxy (eg. propargyloxy), all optionally substituted by $C_{1-4}$ alkyl (eg. methyl), halogen (eg. chlorine, bromine, fluorine or iodine), cyano, or halo $C_{1-4}$ alkyl (eg. trifluoromethyl); or (iii) aryl (eg.

phenyl) optionally substituted by $C_{1-4}$ alkyl (eg. methyl), halogen (eg. chlorine, bromine, fluorine or iodine), $C_{1-4}$ alkoxy (eg. methoxy), nitro, haloalkyl (eg. trifluoromethyl) or cyano; or (iv) a 5- or 6-membered heterocyclyl group, which may be linked through a carbon or a nitrogen atom in the ring, (eg. 2-furyl, 3-furyl, 2-thienyl, 3-thienyl, 1-pyrazolyl, 1-(1,2,4-triazolyl), 2-pyridyl, 3-pyridyl, 4-pyridyl) itself optionally substituted by $C_{1-4}$ alkyl (eg. methyl), halogen (eg. chlorine or bromine), nitro, halo $C_{1-4}$ alkyl (eg. trifluoro-methyl) or cyano; or (v) $C_{1-4}$ alkoxycarbonyl (eg. methoxycarbonyl, ethoxycarbonyl); or (vi) carbamoyl; or (vii) thiocarbamoyl.

Preferably the cyanoalkyl group is unsubstituted and cyanomethyl is especially preferred.

When $R^1$ is an optionally substituted heterocyclyl group, the heterocyclyl group may be, for example, 2-thiazolyl, 2-furyl, 3-isoxazolyl or 1,3,4-thiadiazolyl. Examples of substituents for the heterocyclyl group are $C_{1-4}$ alkyl (eg. methyl), halogen (eg. chlorine, fluorine, bromine or iodine), and $C_{1-4}$ alkoxy (eg. methoxy or ethoxy).

When $R^1$ is an optionally substituted allenylmethyl group the allenylmethyl group may contain 4-10 carbon atoms, but preferably contains 4 carbon atoms. Examples of substituents include one or more $C_{1-4}$ alkyl (eg. methyl, ethyl, propyl or <u>n</u>-, <u>i</u>-, <u>s</u> or <u>t</u>-butyl) and halogen (eg. chlorine, fluorine, bromine or iodine).

When $R^2$ is H or an optionally substituted cyanoalkyl, alkenyl, alkynyl or allenylmethyl group, the definitions of these groups, and their optional substituents, are precisely the same as those given above for $R^1$.

In a further aspect the invention provides compounds having the general formula I wherein R is hydrogen, methyl, ethyl, propyl, butyl, allyl, propargyl, haloallyl, halopropargyl, benzyl, halobenzyl, halo $C_{1-4}$ alkyl benzyl,

$C_{1-4}$ alkoxybenzyl, nitrobenzyl, $C_{3-6}$ cycloalkyl, $C_{3-6}$ cycloalkyl $C_{1-4}$ alkyl, or a metal ion;

$R^1$ is propargyl, halopropargyl, allyl, haloallyl, allenylmethyl, $C_{4-5}$ alkynyl, $C_{1-4}$ alkoxy $C_{1-4}$ alkyl, cyano $C_{1-10}$ alkyl, cyano $C_{3-6}$ cycloalkyl, $C_{1-4}$ alkoxycarbonyl, $C_{1-4}$ alkyl carbamoyl $C_{1-4}$ alkyl, thiocarbamoyl $C_{1-4}$ alkyl, heterocyclyl, heterocyclyl $C_{1-4}$ alkyl, heterocyclyl (cyano) $C_{1-4}$ alkyl, heterocyclyl (thiocarbamoyl) $C_{1-4}$ alkyl, halophenyl (cyano) $C_{1-4}$ alkyl, cyano (carbamoyl) $C_{1-4}$ alkyl, cyano (thiocarbamoyl) $C_{1-4}$ alkyl, cyano ($C_{1-4}$ alkoxy carbonyl) $C_{1-4}$ alkyl, cyano ($C_{1-4}$ alkoxy) $C_{1-4}$ alkyl, $C_{1-4}$ alkylthio $C_{1-4}$ alkyl, halo $C_{1-4}$ alkyl, cyano ($C_{3-4}$ alkynyloxy) $C_{1-4}$ alkyl, cyano ($C_{3-4}$ alkenyloxy) $C_{1-4}$ alkyl; and $R^2$ is hydrogen, $C_{1-4}$ alkyl, allyl, haloallyl, propargyl, halopropargyl, or cyano $C_{1-4}$ alkyl.

In the foregoing paragraph the heterocyclyl function or moiety is preferably

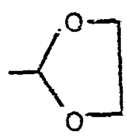 , 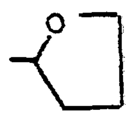 , 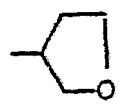 ,

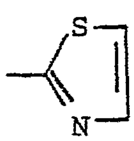 , 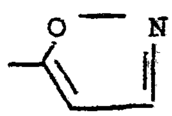 , 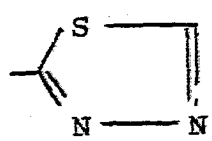 ,

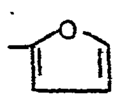 , 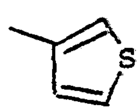 , 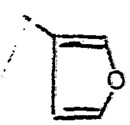 ,

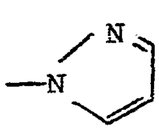 , 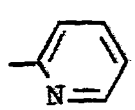 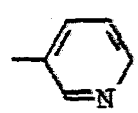

or 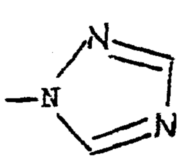

0201999

In a still further aspect the invention provides compounds of formula I wherein R is $C_{1-4}$ alkyl, allyl or propargyl; $R^2$ is hydrogen; and $R^1$ is cyano $C_{1-4}$ alkyl, thiocarbamoyl $C_{1-4}$ alkyl, cyano (furyl) $C_{1-4}$ alkyl or cyano (thienyl) $C_{1-4}$ alkyl.

In a yet further aspect the invention provides compounds of formula 1 wherein R is methyl, ethyl or propargyl; $R^2$ is hydrogen; and $R^1$ is cyano $C_{1-2}$ alkyl, thiocarbamoyl methyl, cyano (2-furyl) methyl, cyano (3-furyl) methyl, cyano (3-thienyl)methyl.

In the foregoing four paragraphs there are recited values for $R^1$ where there is more than one substituent on an alkyl group. In these cases the second substituent group has been placed in brackets after the first substituent.

In particular the invention provides the compounds having the structural formulae :

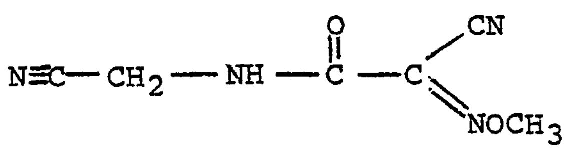

(Compound No. 20 of Table I)

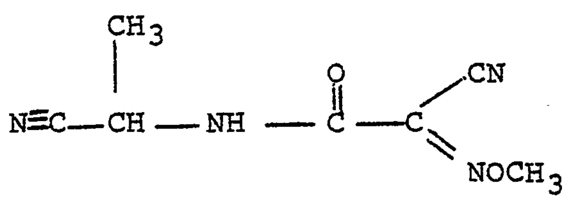

(Compound No. 34 of Table I)

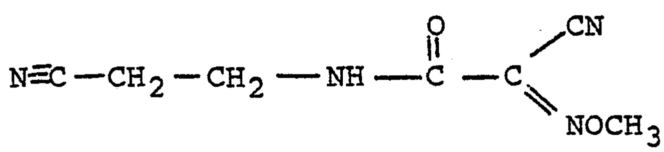

(Compound No. 36 of Table I)

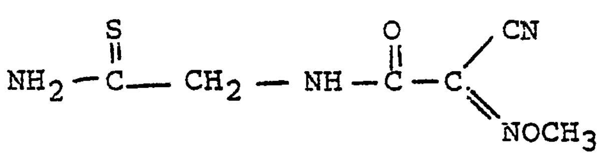

(Compound No. 54 of Table I)

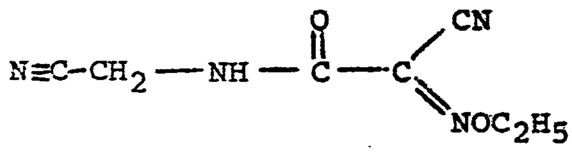

(Compound No. 22 of Table I)

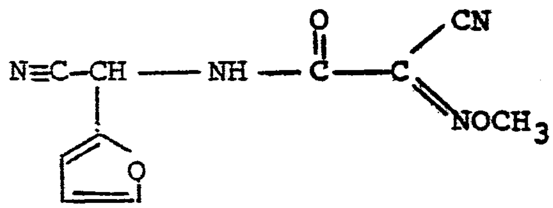

(Compound No. 63 of Table I)

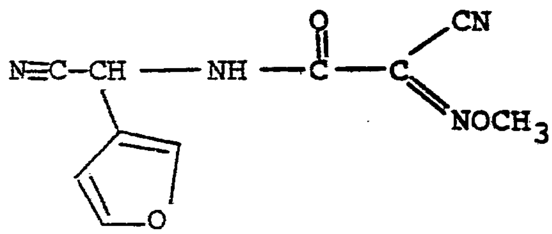

(Compound No. 85 of Table I)

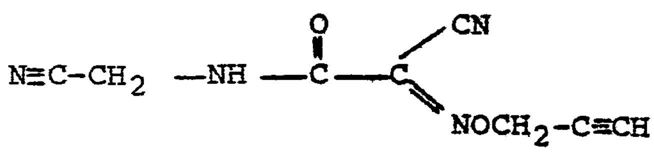

(Compound No. 27 of Table I)

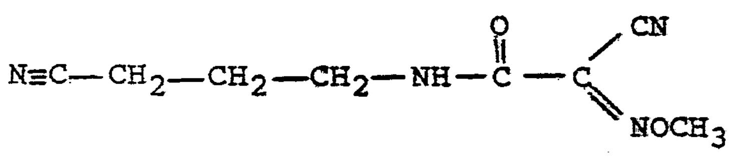

(Compound No. 37 of Table I)

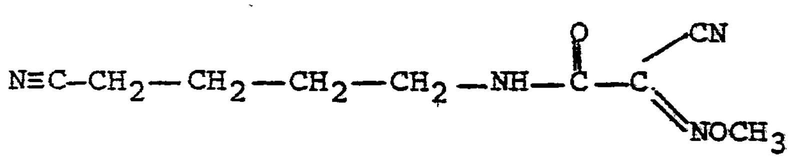

(Compound No. 68 of Table I)

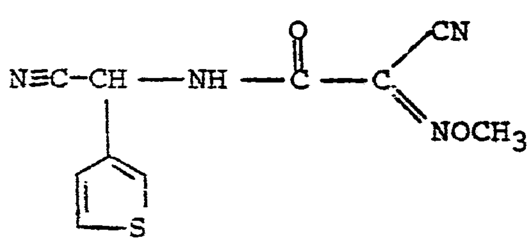

(Compound No. 84 of Table I)

Examples of compounds according to the invention are shown in Table I below in which the different values for R, $R^1$ and $R^2$ are displayed.

TABLE I

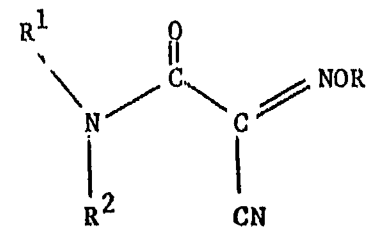

| Compound No. | $R^1$ | $R^2$ | R | Melting Point (°C) | Preparation Method |
|---|---|---|---|---|---|
| 1 | $CH_2C{\equiv}CH$ | H | $CH_3$ | 84-86 | A |
| 2 | $CH_2C{\equiv}CH$ | H | $C_2H_5$ | | |
| 3 | $CH_2C{\equiv}CH$ | H | $CH_2C{\equiv}CH$ | | |
| 4 | $CH_2CH{=}CH_2$ | H | $CH_3$ | 46-48 | A |
| 5 | $CH_2C{\equiv}CI$ | H | $CH_3$ | | |
| 6 | $CH_2C{\equiv}CBr$ | H | $CH_3$ | | |
| 7 | $CH_2CI{=}CHI$ | H | $CH_3$ | 96-98 | F |
| 8 | $CH_2CBr{=}CHBr$ | H | $CH_3$ | oil | F |
| 9 | $CH_2C{\equiv}CH$ | $CH_2C{\equiv}CH$ | $CH_3$ | oil | B |
| 10 | $CH_2C{\equiv}CH$ | $CH_2CH{=}CH_2$ | $CH_3$ | oil | B |
| 11 | $CH_2CI{=}CI_2$ | H | $CH_3$ | | |
| 12 | $CH_2CH{=}C{=}CH_2$ | H | $CH_3$ | 44-46 | J |
| 13 | $CH_2CH_2OC_2H_5$ | H | $CH_3$ | oil | A |
| 14 | $CH_2CH(OCH_3)_2$ | H | $CH_3$ | 48-49 | A |

TABLE I (cont)

| Compound No. | $R^1$ | $R^2$ | R | Melting Point (°C) | Preparation Method |
|---|---|---|---|---|---|
| 15 | $CH_2CH_2OCH_3$ | H | $CH_3$ | 79-80 | A |
| 16 | $CH_2CH(OC_2H_5)_2$ | H | $CH_3$ | 44-46 | A |
| 17 | 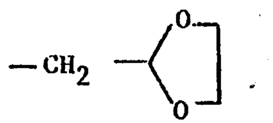 | H | $CH_3$ | 96-98 | A |
| 18 | 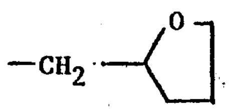 | H | $CH_2$ | 28-30 | A |
| 19 | 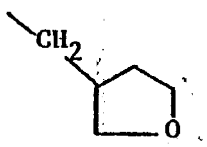 | H | $CH_3$ | 74-75 | A |
| 20 | $CH_2CN$ | H | $CH_3$ | 83-85 | B |
| 21 | $CH_2CN$ | $CH_2C\equiv CH$ | $CH_3$ | | |

TABLE I (cont)

| Compound No. | $R^1$ | $R^2$ | R | Melting Point (°C) | Preparation Method |
|---|---|---|---|---|---|
| 22 | $CH_2CN$ | H | $C_2H_5$ | 73-75 | B |
| 23 | $CH_2CONH_2$ | H | $CH_3$ | 169-171 | B |
| 24 | $CH_2COOCH_3$ | H | $CH_3$ | 65-67 | B |
| 25 | 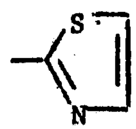 | H | $CH_3$ | 203-205 | C |
| 26 | 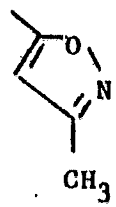 | H | $CH_3$ | 167-168 | C |
| 27 | $CH_2CN$ | H | $CH_2C{\equiv}CH$ | 57-60 | E |
| 28 | $CH_2CN$ | H | $CH_2CH{=}CH_2$ | oil | B |
| 29 | $CH_2CN$ | H | $CH_2C_6H_5$ | 108-111 | E |
| 30 | $CH_2CN$ | H | $CH_2C{\equiv}C-I$ | | |
| 31 | $CH_2CN$ | H | H | 144-146 | E |
| 32 | $CH_2CN$ | $CH_2CH{=}CH_2$ | $CH_3$ | | |

TABLE I (cont)

| Compound No. | $R^1$ | $R^2$ | R | Melting Point (°C) | Preparation Method |
|---|---|---|---|---|---|
| 33 | $CH_2CN$ | $CH_3$ | $CH_3$ | 88-90 | B |
| 34 | $CH(CH_3)CN$ | H | $CH_3$ | Oil | B |
| 35 | $C(CH_3)_2CN$ | H | $CH_3$ | 139-140.5 | B |
| 36 | $CH_2CH_2CN$ | H | $CH_3$ | 80-81 | B |
| 37 | $CH_2CH_2CH_2CN$ | H | $CH_3$ | 58-61 | B |
| 38 | $CH_2C\equiv CH$ | $CH_3$ | $CH_3$ | Oil | B |
| 39 | $C(CH_3)_2C\equiv CH$ | H | $CH_3$ | 46-48 | B |
| 40 | $CH_2C\equiv CH$ | H | H | | |
| 41 | $CH_2C\equiv CH$ | H | Cu | | |
| 42 | $CH_2C\equiv CH$ | H | Na | | |
| 43 | $CH_2CN$ | H | Cu | >300 | K |
| 44 | $CH_2CN$ | H | Na | | |
| 45 | $CH_2CN$ | H | Fe | >300 | K |
| 46 | $CH_2CN$ | $CH_2C\equiv C-I$ | $CH_3$ | | |
| 47 | $CH_2C\equiv C-I$ | $CH_2C\equiv C-I$ | $CH_3$ | | |
| 48 | $CH_2CN$ | $CH_2CI=CHI$ | $CH_3$ | | |
| 49 | $CH_2CN$ | $CH_2CBr=CHBr$ | $CH_3$ | | |

TABLE I (cont)

| Compound No. | $R^1$ | $R^2$ | R | Melting Point (°C) | Preparation Method |
|---|---|---|---|---|---|
| 50 | 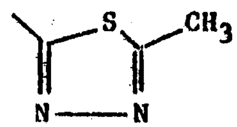 | H | $CH_3$ | 215-217 | C |
| 51 | $CH_2CN$ | $CH_2CN$ | $CH_3$ | 138-139.5 | B |
| 52 | $CH_2CN$ | $CH_2CN$ | H | | |
| 53 | $CH_2CN$ | $CH_2CH_2CN$ | $CH_3$ | 98-99 | B |
| 54 | $CH_2CSNH_2$ | H | $CH_3$ | 120-121 | |
| 55 | $CH_2CSNH_2$ | H | $C_2H_5$ | 142-143 | H |
| 56 | $CH_2CSNH_2$ | $CH_2C{\equiv}CH$ | $CH_3$ | | |
| 57 | $(CH_2)_5CN$ | H | $CH_3$ | Oil | B |
| 58 | $CH_2CN$ | H | $4\text{-}Cl\text{-}C_6H_4CH_2$ | | |
| 59 | $CH_2CN$ | H | $4\text{-}CH_3O\text{-}C_6H_4CH_2$ | | |
| 60 | $CH_2CN$ | H | $4\text{-}CF_3\text{-}C_6H_4CH_2$ | | |
| 61 | $CH_2CN$ | H | $2,4\text{-}diCl\text{-}C_6H_3CH_2$ | | |
| 62 | $CH_2CN$ | H | $2\text{-}NO_2\text{-}C_6H_4CH_2$ | | |
| 63 | 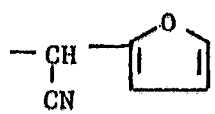 | H | $CH_3$ | Oil | B |

TABLE I (cont)

| Compound No. | $R^1$ | $R^2$ | R | Melting Point (°C) | Preparation Method |
|---|---|---|---|---|---|
| 64 | $CH_2CN$ | H | $CH_2CH_2CH_3$ | Oil | E |
| 65 | $CH_2CN$ | H | $CH(CH_3)_2$ | 65-69 | E |
| 66 | $CH_2CN$ | H | $-C(CH_3)_3$ | | |
| 67 | $CH_2CN$ | H | $(CH_2)_{11}CH_3$ | 76-78 | E |
| 68 | $(CH_2)_4CN$ | H | $CH_3$ | Oil | B |
| 69 | $(CH_2)_6CN$ | H | $CH_3$ | | |
| 70 | $(CH_2)_7CN$ | H | $CH_3$ | | |
| 71 | $(CH_2)_8CN$ | H | $CH_3$ | Oil | B |
| 72 | $(CH_2)_9CN$ | H | $CH_3$ | | |
| 73 | $CH(C_2H_5)CN$ | H | $CH_3$ | Oil | B |
| 74 | $CH(iso\text{-}C_3H_7)CN$ | H | $CH_3$ | 98-101 | B |
| 75 | $CH(cyclo\text{-}C_6H_{11})CN$ | H | $CH_3$ | 97-100 | B |
| 76 | $CH_2CN$ | H | $CH_2$– 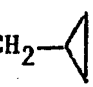 | Oil | E |
| 77 | $CH_2CN$ | H | Cyclopentyl | Oil | E |
| 78 | $CH_2CN$ | H | $CH_2CH=CHCl$ (E isomer) | Oil | E |

TABLE I (cont)

| Compound No. | $R^1$ | $R^2$ | R | Melting Point (°C) | Preparation Method |
|---|---|---|---|---|---|
| 79 | $CH_2CH_2CSNH_2$ | H | $CH_3$ | 128 (dec) | H |
| 80 | $CH(CH_3)CSNH_2$ | H | $CH_3$ | 140 (dec) | H |
| 81 | $CH_2CH_2CN$ | H | $C_2H_5$ | 83-84 | B |
| 82 | 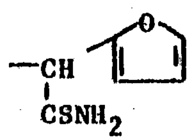 | H | $CH_3$ | | |
| 83 | 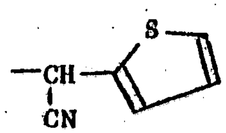 | H | $CH_3$ | Oil | B |
| 84 | 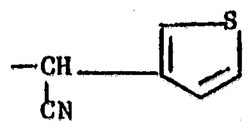 | H | $CH_3$ | Oil | B |
| 85 | 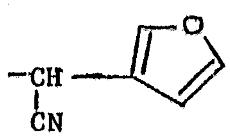 | H | $CH_3$ | 86-88 | B |
| 86 | 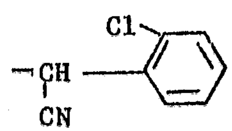 | H | $CH_3$ | 110-112 | B |

TABLE I (cont)

| Compound No. | $R^1$ | $R^2$ | R | Melting Point (°C) | Preparation Method |
|---|---|---|---|---|---|
| 87 | 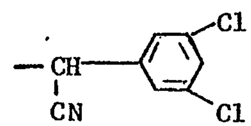 | H | $CH_3$ | 132-133 | B |
| 88 | 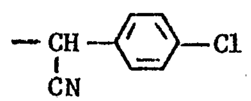 | H | $CH_3$ | 113-115 | B |
| 89 | 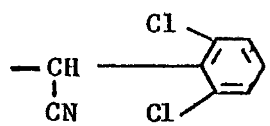 | H | $CH_3$ | 111-113 | B |
| 90 | 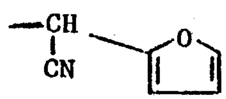 | H | $C_2H_5$ | 65-68 | B |
| 91 | $CH(CH_3)CN$ | H | $C_2H_5$ | 61-63 | B |

TABLE I (cont)

| Compound No. | $R^1$ | $R^2$ | R | Melting Point (°C) | Preparation Method |
|---|---|---|---|---|---|
| 92 | $CH(CONH_2)CN$ | H | $CH_3$ | foam | B |
| 93 | $CH(COOC_2H_5)CN$ | H | $CH_3$ | 84-85 | B |
| 94 | $CH(CSNH_2)CN$ | H | $CH_3$ | | |
| 95 | 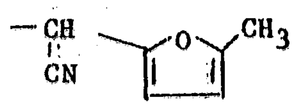 | H | $CH_3$ | Oil | B |
| 96 | $CH(OCH_3)CN$ | H | $CH_3$ | 70-74 | I |
| 97 | $CH(OC_2H_5)CN$ | H | $CH_3$ | Oil | I |
| 98 | 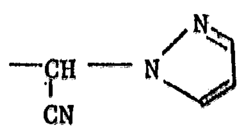 | H | $CH_3$ | 167 (dec.) | I |
| 99 | 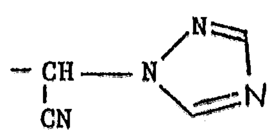 | H | $CH_3$ | 170-177 | I |

TABLE I (cont)

| Compound No. | $R^1$ | $R^2$ | R | Melting Point (°C) | Preparation Method |
|---|---|---|---|---|---|
| 100 | $CH_2CH_2SCH_3$ | H | $CH_3$ | Oil | B |
| 101 | $CH_2CH_2Cl$ | H | $CH_3$ | 65-69 | B |
| 102 | 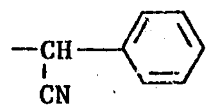 | H | $CH_3$ | Oil | B |
| 103 | $CH_2CH_2CN$ | $CH_2CH_2CN$ | $CH_3$ | 99-101 | B |
| 104 | $CH(OCH_2C{\equiv}CH)CN$ | H | $CH_3$ | Oil | I |
| 105 | 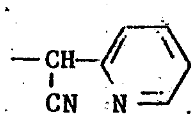 | H | $CH_3$ | 135-137 | B |
| 106 | 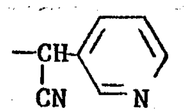 | H | $CH_3$ | 140-141 | B |

For certain compounds which are characterised as oils in Table I, proton NMR data is provided, and is shown in Table II. Chemical shifts are measured in ppm from tetramethylsilane internal standard, and deuterochloroform was used as the solvent throughout. The following abbreviations are used :

b = broad
s = singlet
d = doublet
t = triplet
q = quartet
m = multiplet

TABLE II

| Compound No. | Proton NMR data (ppm, $CDCl_3$) |
|---|---|
| 8 | 4.24 (s,3H); 4.38 (d,2H); 6.60 (s,1H); 7.15 (bs,1H) |
| 9 | 2.32 (bs,2H); 4.20 (s,3H); 4.38 (bs,4H) |
| 10 | 2.30 (bs,1H); 4.10-4.30 (m,7H); 5.20 (d,1H); 5.36 (s,1H); 5.60-6.05 (m,1H) |
| 28 | 4.25 (d,2H); 4.92 (d,2H); 5.28 (s,1H): 5.42 (d,1H); 5.72-6.20 (m,1H); 7.35 (bs,1H) |
| 34 | 1.38 (d,3H); 4.18 (s,3H); 4.90 (q,1H); 9.40 (bs,1H) |
| 38 | 2.25-2.45 (m,1H); 3.12 (s,0.3H); 3.25 (s,0.7,H); 4.24 (s,3H); 4.35 (d,2H) |

TABLE II (cont)

| Compound No. | Proton NMR data (ppm, $CDCl_3$) |
|---|---|
| 57 | 1.44 -1.80 (m,6H); 2.40 (t,2H); 3.40 (q,2H); 4.26 (s,3H); 6.80 (bs,1H) |
| 63 | 4.32 (s,3H); 6.22 (d,1H); 6.40-6.48 (m,1H); 6.64 (d,1H); 7.32 (bd,1H); 7.50 (d,1H) |
| 64 | 1.42 (t,3H); 4.32 (bd,2H); 4.76 (septet, 1H); 7.36 (bd,1H) |
| 68 | 1.60-1.90 (m,4H); 2.35-2.56 (m,2H); 3.32-3.60 (m,2H); 4.32 (s,3H); 6.65 (bs,1H) |
| 71 | 1.20-1.80 (m,12H); 2.35 (t,2H); 3.36 (q,2H); 4.24 (s,3H) |
| 73 | 1.13 (t,3H); 1.95 (quintet, 2H); 4.31 (s,3H); 4.88 (q,1H); 6.98 (bd,1H) |
| 76 | 0.30-0.48 (m,2H); 0.50-0.80 (m,2H); 1.05-1.25 (m,1H); 4.30 (d,4H); 7.30 (bs,1H) |
| 77 | 1.50-2.10 (m,8H); 4.32 (d,2H); 5.04 (m,1H); 7.26 (m,1H) |
| 78 | 4.32 (d,2H); 4.92 (d,2H); 5.98-6.28 (m,1H); 6.52 (d,1H); 7.25 (m,1H) |

TABLE II (cont)

| Compound No. | Proton NMR data (ppm, $CDCl_3$) |
|---|---|
| 83 | 4.18 (s,3H); 6.50 (bs,1H); 7.0 (m,1H); 7.20 (d,1H); 7.55 (d,1H); 10.12 (bs,1H) |
| 84 | 4.24 (s,3H); 6.15 (d,1H); 6.95 (bd,1H); 7.06 (dd,1H); 7.32-7.44 (m,1H); 7.48-7.62 (m,1H) |
| 92* | 4.22 (s,3H); 5.55 (s,1H); 7.66 (bd,2H); 9.62 (bs,1H) |
| 95 | 2.22 (s,3H); 4.20 (s,3H); 6.05 (bd,1H); 6.28 (bd,1H); 6.42 (d,1H); 9.95 (bd,1H) |
| 97 | 1.29 (t,3H); 3.74 (q,2H); 4.36 (s,3H); 6.08 (d,1H); 7.52 (bd,1H) |
| 100 | 2.11 (s,3H); 2.68 (t,2H); 3.55 (q,2H); 4.22 (s,3H); 7.00 (bs,1H) |
| 102 | 4.22 (s,3H); 6.14 (d,1H); 7.04 (bd,1H); 7.44 (m,5H) |
| 104 | 2.64 (t,1H); 4.32 (s,3H); 4.35 (d,2H); 6.8 (d,1H); 7.52 (bd,1H) |

TABLE II (cont)

| Compound No. | Proton NMR data (ppm, $CDCl_3$) |
|---|---|
| 105+ | 4.26 (s,3H); 6.60 (d,1H); 7.44 (dd,1H); 7.55 (d,1H); 7.88 (t,1H); 8.52 (bs,1H); 8.64 (d,1H) |

* spectrum run in DMSO-$d^6$

\+ spectrum run in $CD_3CN$

Preferred compounds of Table I are those numbered 20, 34, 36, 54, 22, 63, 85. Also those numbered 27, 37, 68 and 84.

In the structure

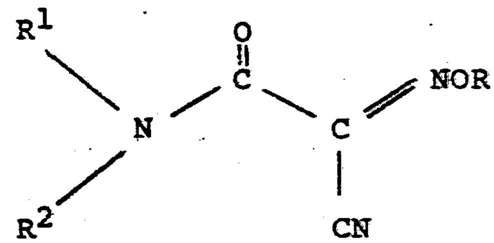

the oxime can exist in stereoisomeric forms E and Z

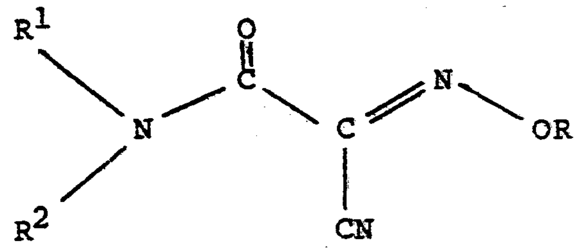

E

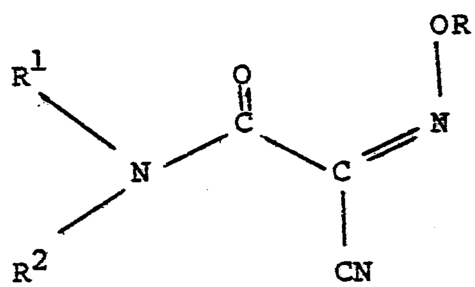

Z

This invention therefore embraces the stereoisomers. The invention compounds may be prepared by several routes, (A) to (K), described below, and each of these processes is to be considered to be constituting part of the present invention.

Route A

A compound of formula (I) may be produced by reacting an ester of formula (III) with an amine $R^1R^2NH$

in a convenient solvent such as ethanol, methanol, acetonitrile or dimethylformamide (DMF) at 0°-200°C.

The product can be isolated by evaporating the solvent and treating the residue with water and either recrystallising the solid formed from a convenient solvent, or extracting the aqueous phase with a convenient solvent such as diethyl ether or ethyl acetate, and evaporating the solvent to give the product. Purification can be effected by chromatography, or recrystallisation from a suitable solvent.

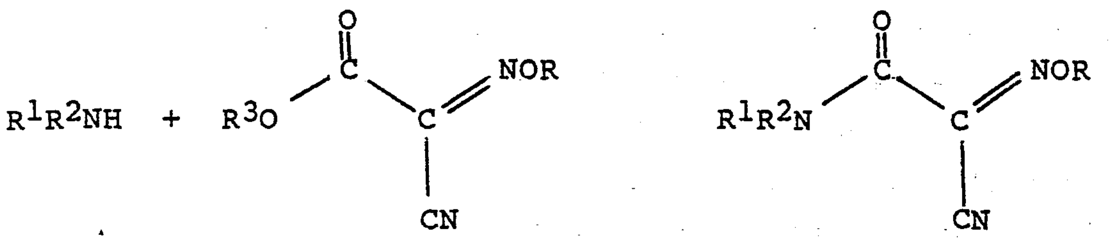

(III) (I)

$R^3$ is alkyl containing from 1 to 10 carbon atoms.

The esters of formula (III) may be produced by alkylation of the known oxime of formula (II). The alkylation may be performed by first treating the oxime (II) with a base such as an alkali metal hydroxide (eg. sodium hydroxide), alkali metal alkoxide (eg. sodium ethoxide), alkali metal carbonate (eg. potassium carbonate) or a tertiary amine (eg. triethylamine, but preferably with sodium hydroxide, in a suitable solvent such as water, alkanols (eg. ethanol or methanol), ketones (eg. methyl ethyl ketone or acetone), acetonitrile, dimethylformamide, tetrahydrofuran, or mixtures thereof, but preferably a mixture of water and methyl ethyl ketone. The oxime anion so generated is then treated with a halide RX where R is defined as in formula (I) and X is chlorine, bromine, iodine, or methane- or p-toluene-sulphonate, or with a dialkylsulphate such as dimethylsulphate,

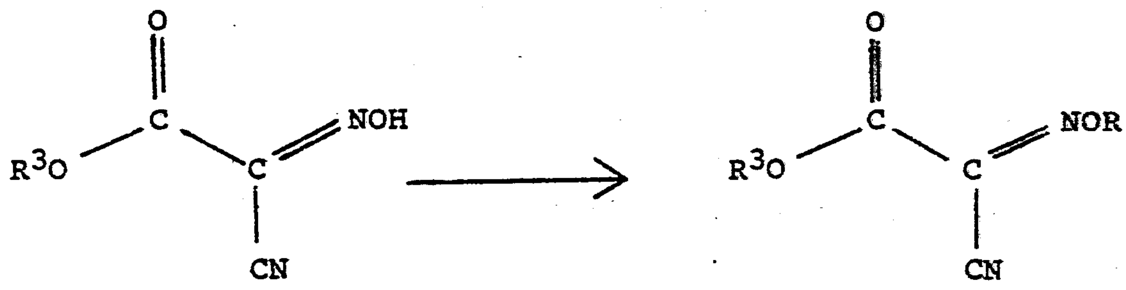

(II) (III)

at 0°-200°C. The product (III) is isolated by extraction with a suitable solvent such as ethyl acetate, methylene chloride or diethyl ether and evaporating the solvent. The product, especially where $R^3$ is ethyl and R is methyl can be purified by distillation at reduced pressure, or by crystallisation at -60° to 0°C, but especially at -45 to -30°, from a suitable solvent such as a lower alkanol (eg. ethanol or methanol), to provide (III) as a solid.

<u>Route B</u>

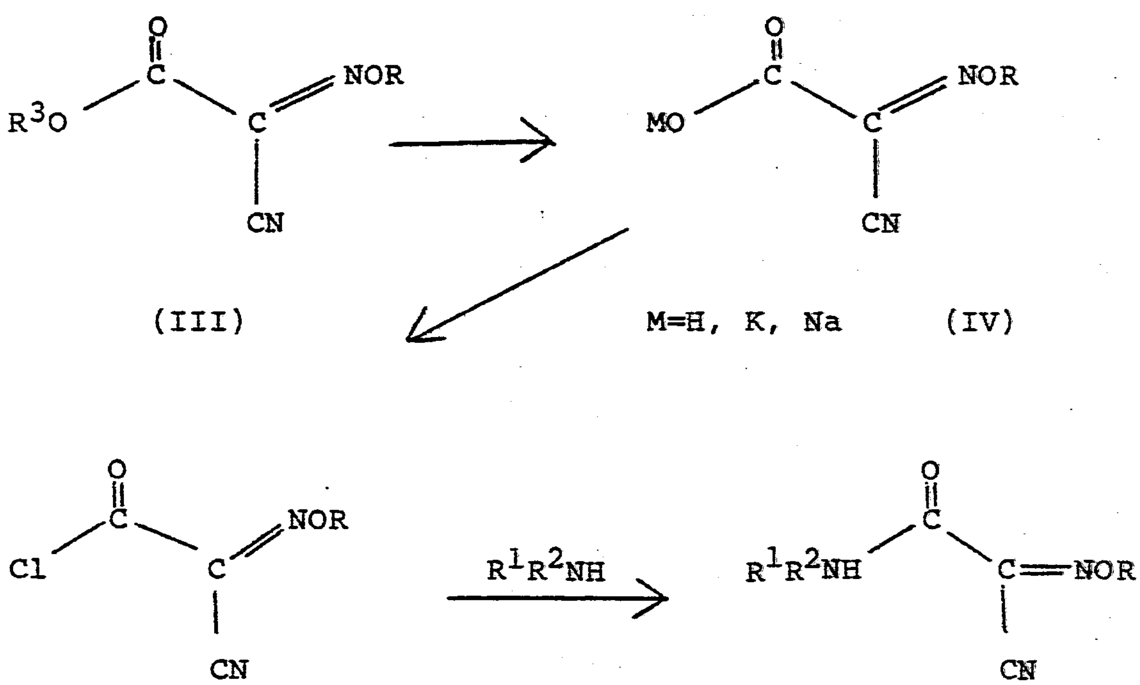


(III) M=H, K, Na (IV)

(V) (I)

In Route B compounds of formula (I) can be prepared by reacting an acid chloride of formula (V) with an amine $R^1R^2NH$ in a convenient solvent such as toluene, ether or ethyl acetate at 0°-100°C, in the presence of an acid binding agent such as triethylamine, pyridine or potassium carbonate. The reaction can also be carried out in a two phase system such as toluene and water where the amine component is not soluble in common organic solvents, in which case a solution of the acid chloride in a convenient solvent such as toluene or ethyl acetate is added to the amine in water containing an acid binding agent.

The product (I) can be isolated by pouring the mixture into water and extracting with a convenient organic solvent in the usual way.

The acid chloride (V) can be produced by reacting the acid or its dry salt (IV) with a reagent such as thionyl chloride or oxalyl chloride in a convenient solvent such as toluene, ether or chloroform containing a trace of DMF. The acid chloride (V), especially where R is methyl, can be purified by distillation.

The acid or its salt (IV) can be produced by hydrolysing the ester of formula (III) with an alkali metal hydroxide in a convenient solvent such as ethanol, methanol or water at 20-100°C.

When $R^1R^2NH$ was an $\alpha$-amino nitrile that was not commercially available it was prepared using the standard Strecker reaction conditions of reaction of an aldehyde with ammonium chloride and a metal cyanide, followed by additional treatment with ammonia gas in some cases. Other amino nitriles of general structure $NC(CH_2)_nNH_2$, where n is 3-10, were prepared, when not commercially available, by the standard Gabriel synthesis, using potassium phthalimide and the haloalkylcyanide, followed by cleavage of the cyanoalkyl phthalimide with hydrazine.

Route C

In route C compounds of formula (I) can be prepared by reacting compounds of formula (VI) with a strong base such as sodium hydride or lithium diisopropylamide in a convenient solvent such as dimethylformamide (DMF), tetrahydrofuran (THF) of dimethoxyethane (DME) at -50°-100°C, and reaction with an alkyl halide $R^2X$ (VII) <u>in situ</u> or by addition

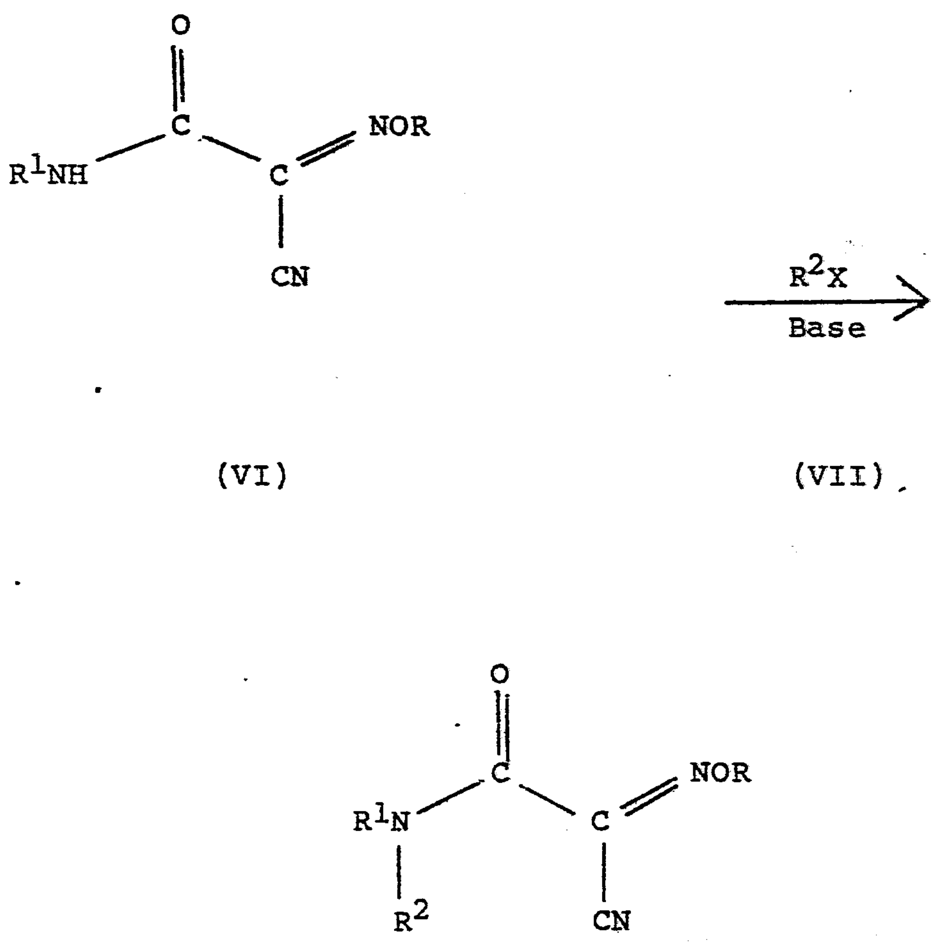


(VI)

(VII).

(I)

afterwards. $R^2$ is as defined in the generic formula (I). The reaction can be worked up as for B.

Route D

In route D compounds of formula (I) can be produced by reacting an ester of formula (III) with an amine anion of formula (IX) where $R^4$ is a heterocyclyl radical such as 2-thiazolyl, 2-(5-alkyl-1,3,4-thiadiazolyl) or 5-(3-alkylisoxazolyl) in a convenient solvent such as DMF, DME or THF at -50°-100°C, and quenching the reaction into aqueous mineral acid and extracting the product (X) with a convenient solvent in the usual way.

The anion can be generated by treatment of an amine of formula (VIII) with a strong base such as sodium hydride or lithium diisopropylamide.

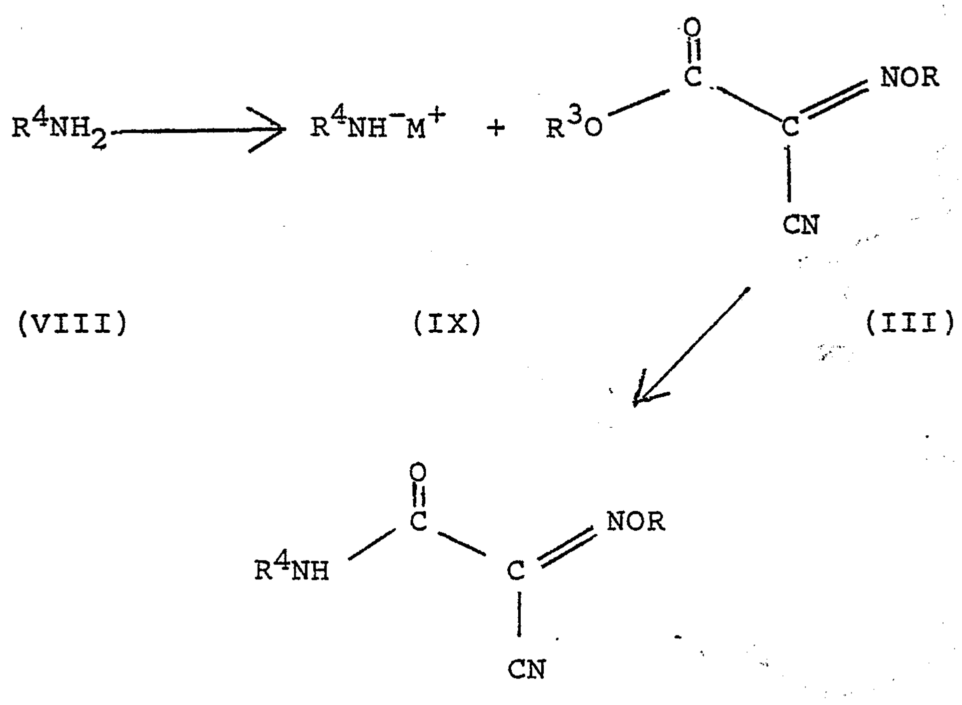

(VIII) (IX) (III)

(X)

Route E

In route E a compound of formula (I) may be produced by reacting an oxime of formula (XI) with a base such as a tertiary amine, metal alkoxide or metal carbonate in a convenient solvent such as acetone, ethanol, acetonitrile, or DMF at 0°-200°C, followed by a compound RX where R is as defined in the generic formula (I) and X can be a halogen such as chlorine, bromine or iodine, or a sulphonate such as p-toluenesulphonate or by a dialkylsulphate such as dimethylsulphate. The product can be isolated by pouring into water and crystallisation from a convenient solvent or by solvent extraction.

The oxime (XI) can be produced by reaction of the amide (XII)

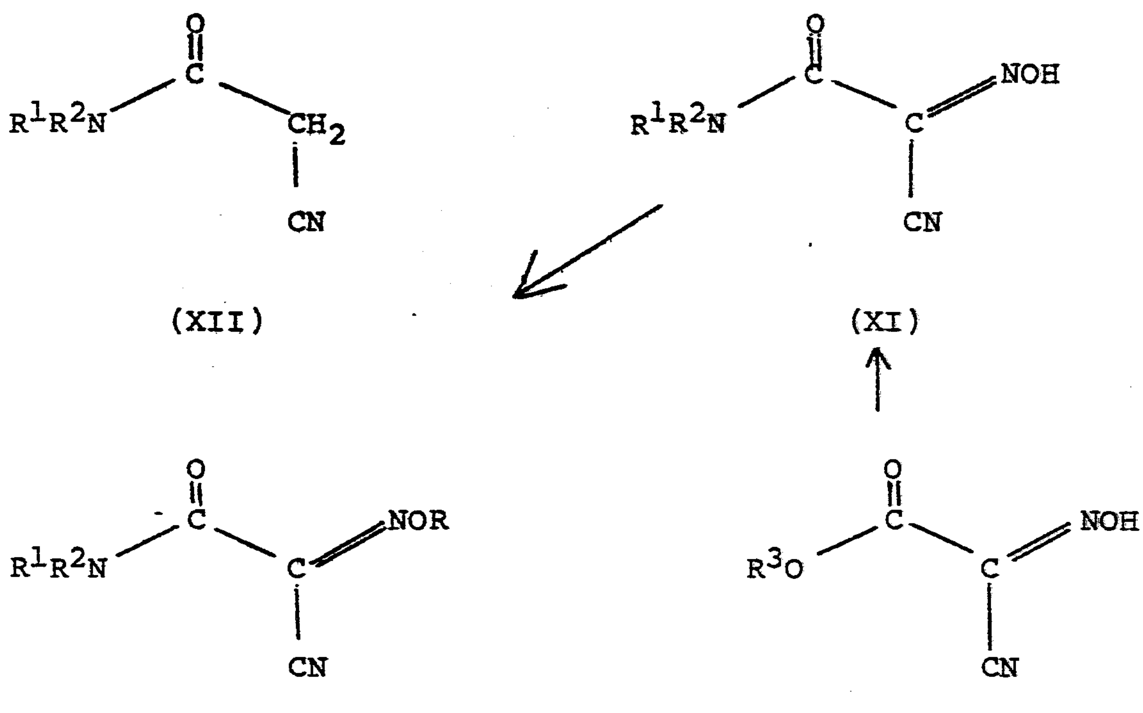

(XII) (XI)

(I) (II)

with a nitrite ester (such as amyl nitrite) and a base such as a metal alkoxide (such as sodium ethoxide) or sodium hydride in a convenient solvent such as ethanol, DMF or acetonitrile, or with a metal nitrite (such as sodium nitrite) in a convenient solvent such as water, containing an acid such as acetic acid or hydrochloric acid.

Alternatively the oxime (XI) can be produced by reaction of an ester of formula (II) with an amine $R^1R^2NH$ in a convenient solvent such as ethanol, DMF, acetonitrile or DME, at -20°C-200°C.

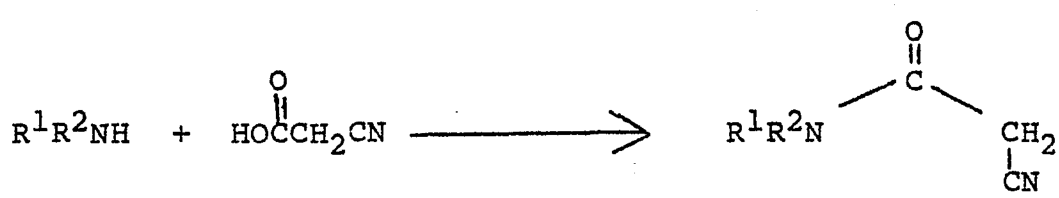

(XII)

The amide (XII) can be produced by condensation of an amine $R^1R^2NH$ with cyanoacetic acid, by (i) reaction in the presence of a condensing agent such as dicyclohexylcarbodiimide in a suitable solvent such as acetonitrile, ethyl acetate, or methylene chloride at -20°-100°C, preferably in the presence of a catalytic quantity of a pyridine derivative such as 4-dimethylaminopyridine, or (ii) by reaction of the amine $R^1R^2NH$ with an activated derivative of cyanoacetic acid such as cyanoacetyl chloride or a mixed anhydride of cyanoacetic acid (eg. with acetic anhydride) in a suitable solvent such as ethyl acetate or dichloromethane or a two phase system such as ethyl acetate and water, at -20°-100°C.

<u>Route F</u>

In route F, a compound of formula (XIII) where $R^1$ and/or $R^2$ is dihaloalkyl or dihaloalkenyl where the halogens are on adjacent carbon atoms may be produced by reacting a

compound of formula (I) where $R^1$ is alkenyl or alkynyl and $R^2$ is H, alkenyl or alkynyl, with a halogen $X_2$ where X is chlorine, bromine or iodine is a convenient solvent such as chloroform or methylene chloride at -50°-100°C.

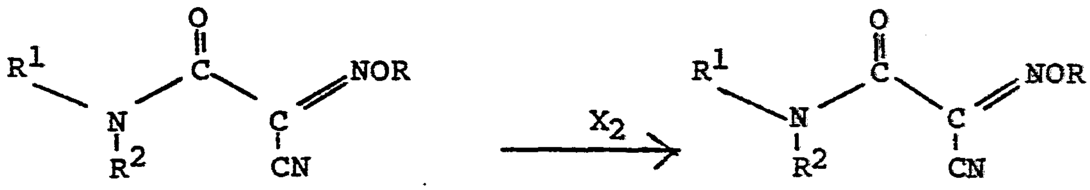


(I) (XIII)

where $R^1$ and/or $R^2$
= dihaloalkyl or dihaloalkenyl.

The halogen atoms in the product may be cis or trans to each other.

Route G

The compounds of general formula (XIV), where $R^1$ is a substituted alkyl group where at least one substituent is thiocarbamoyl and $R^2$ is as defined in general formula (I) may be produced by treatment of compounds of general formula (I) where $R^1$ is a cyanoalkyl group, with hydrogen sulphide gas in the presence of an amine such as triethylamine or ammonia, in a suitable solvent such as pyridine or toluene.

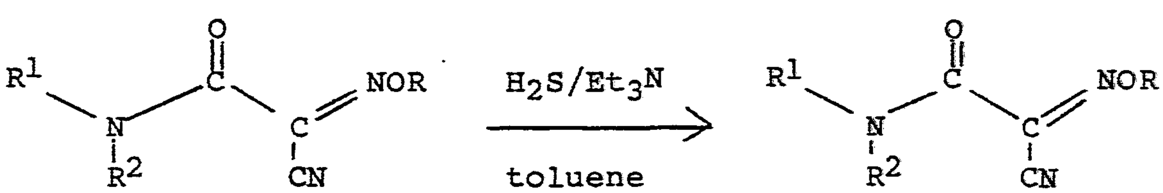


(I) (XIV)

$R^1$=cyanoalkyl $R^1$=thiocarbamoylalkyl

However this method tends to produce impure material which requires extensive purification, due to concomitant reduction of the oxime function under the reaction conditions. An alternative method is described in Route H.

Route H

In Route H, a compound of general formula (XIV) where $R^1$ is a substituted alkyl group, where at least one substituent is thiocarbamoyl, and $R^2$ is as defined in the general formula (I) may be produced in several steps.

Step 1

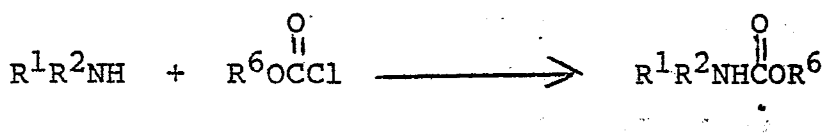

(XV)

$R^1$=carbamoylalkyl $R^1$=carbamoylalkyl

An amide of formula (XV), where $R^1$ is carbamoylalkyl and $R^2$ is as defined in general formula (I) may be produced by reaction of an amine $R^1R^2NH$, with identical values for $R^1$ and $R^2$, with an acid chloride of formula $R^6OCOCl$ where $R^6$ is an optionally substituted benzyl group (eg. benzyl) or $C_1$-$C_4$ alkyl group (eg. t-butyl) in a suitable solvent such as toluene or ethyl acetate, in the presence of an acid acceptor such as a metal carbonate (eg. sodium bicarbonate) or metal hydroxide (eg. sodium hydroxide) or tertiary amine (eg. triethylamine) at -20°-150°C (preferably room temperature). Alternatively a two-phase system can be used, where the acid chloride $R^6OCOCl$ in a suitable organic solvent such as ethyl acetate or toluene, may be added to the amine $R^1R^2NH$ or a suitable salt thereof, in water in the presence of an acid acceptor such as those previously mentioned.

Step 2

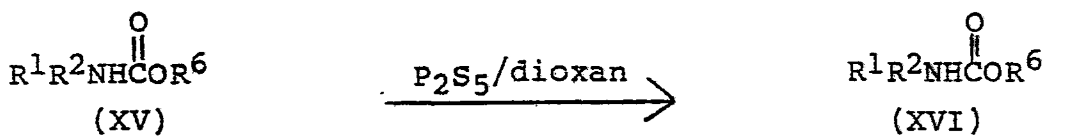


(XV) (XVI)

$R^1$=carbamoylalkyl $R^1$=thiocarbamoylalkyl

The thioamide of formula (XVI), where $R^1$ is an alkyl group substituted by at least one thiocarbamoyl group and $R^2$ is as defined in general formula (I) may be produced by reaction of an amide of formula (XV) with a thionation agent such as phosphorous pentasulphide or Lawesson's reagent in a suitable solvent such as toluene or pyridine or 1,4-dioxan at a temperature of -0°-200°C (preferably 20-100°C), followed by pouring into water and isolation by filtration or solvent extraction in the usual way.

Step 3

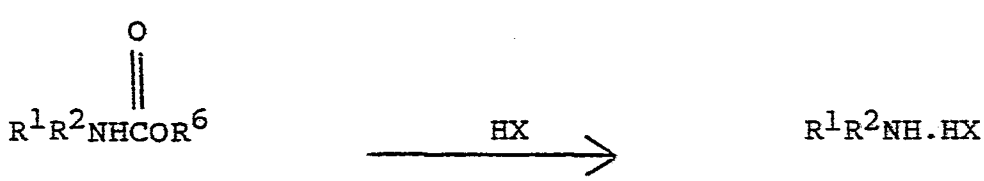


(XVI) (XVII)

$R^1$=thiocarbamoylalkyl $R^1$=thiocarbamoylalkyl

A thioamide of formula (XVII), where $R^1$ is an alkyl group substituted by at least one thiocarbamoyl group and $R^2$ is as defined in general formula (I) may be produced as its hydrogen halide salt, by reaction of a thioamide of formula (XVI) with a hydrogen halide, HX, where X is chloride or bromide, (eg. hydrogen bromide) in a suitable solvent such as acetic acid, at -20°-100°C (preferably at room temperature), and precipitating the hydrogen halide salt of (XVII) by adding a less polar solvent such as diethyl ether, or petroleum ether, and washing the filtered solid product well with the same solvent. The product may conveniently be used without further purification, for Step 4.

Step 4

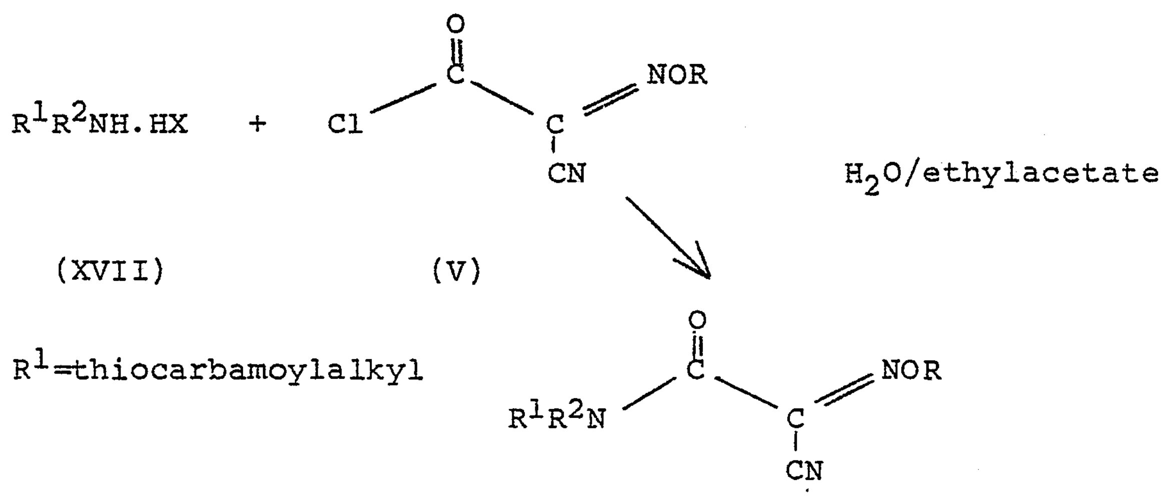


(XVII) (V)

$R^1$=thiocarbamoylalkyl

(I) (XIV)

$R^1$=thiocarbamoylalkyl

A compound of general formula (XIV) where $R^1$ is a substituted alkyl group where at least one substituent is thiocarbamoyl, and $R^2$ is as defined in the general formula (I), may be produced by reaction of the hydrogen halide

salt of the amine $R^1R^2NH$ (XVII) with the acid chloride (V) in a suitable organic solvent such as toluene, ethyl acetate or methylene chloride in the presence of an acid acceptor such as a metal carbonate (eg. potassium carbonate) or tertiary amine (eg. trimethylamine). Alternatively a two-phase in a suitable solvent such as toluene or ethyl acetate is added to the salt of (XVII) in water containing an acid binding agent such as a metal hydroxide, or metal carbonate (eg. sodium hydroxide or potassium carbonate) at 0°-100°C but preferably at 0°-50°C. The product (XIV) can be isolated by pouring the mixture into water and filtration, or by extraction with a convenient organic solvent).

The thioamide of formula (XVI) may also be produced by an alternative method in two steps.

<u>Step 1</u>

$$R^1R^2NH \quad + \quad R^6O\overset{O}{\overset{\|}{C}}Cl \longrightarrow R^1R^2NH\overset{O}{\overset{\|}{C}}OR^6$$

(XVIII) (XIX)

$R^1$=cyanoalkyl $R^1$=cyanoalkyl

An amide of formula (XIX), where $R^1$ is a cyanoalkyl group and $R^3$ is as defined in general formula (I) may be produced by reaction of an amine $R^1R^2NH$ (XVIII) (with the same definitions of $R^1$ and $R^2$) with an acid chloride of formula $R^6OCOCl$ where $R^6$ is as previously defined, in a suitable solvent such as ethyl acetate or toluene. Alternatively a two-phase system may be used whereby the acid chloride $R^6OCOCl$ in a suitable solvent such as ethyl acetate or toluene is added to the salt of (XVIII) in

water containing an acid binding agent such as a metal hydroxide, (eg. sodium hydroxide) or metal carbonate (eg. potassium carbonate) at 0°-100°C, but preferably at 0°-50°C.

The product (XIX) may be isolated by filtration or by extraction with a convenient organic solvent.

Step 2

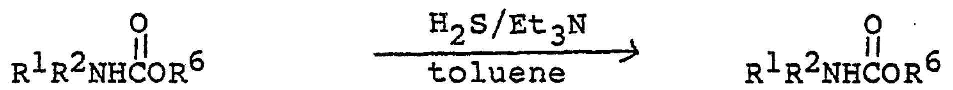


(XIX) (XVI)

$R^1$=cyanoalkyl $R^1$=thiocarbamoylalkyl

The thioamide of formula (XVI) may be produced by reaction of an amide of formula (XIX), where $R^1$ is an optionally substituted cyanoalkyl group and $R^2$ is as defined above, with hydrogen sulphide gas in a suitable solvent such as toluene or pyridine containing a tertiary amine such as triethylamine, -20°-150°C, but preferably at 0°-50°C. The product may be isolated by filtration, or by pouring into water and extraction with a convenient organic solvent in the usual way.

Route I

In route I a compound of formula (XX) where $R^5$ is $C_{1-4}$ alkoxy group, $C_{3-5}$ alkenyloxy group, a $C_{3-5}$ alkynyloxy group, or a 5-membered heterocyclic ring containing two to four nitrogen atoms (eg. pyrazole or 1,2,4-triazole), may be produced in two steps.

Bromination (eg. with bromine $Br_2$) or chlorination (eg. with sulphuryl chloride $SO_2Cl_2$) of (XXI), where R is as defined in general formula (I), in an aprotic solvent such as a lower alkane carboxylic acid ester (eg. ethyl

acetate) or a chlorinated hydrocarbon (eg. chloroform), at -20°C to 100°C, but preferably at 20-50°C, gives the highly reactive bromo- or chloro-nitrile (XXII). (XXII) is too unstable to be isolated and characterised and is used immediately for the second step.

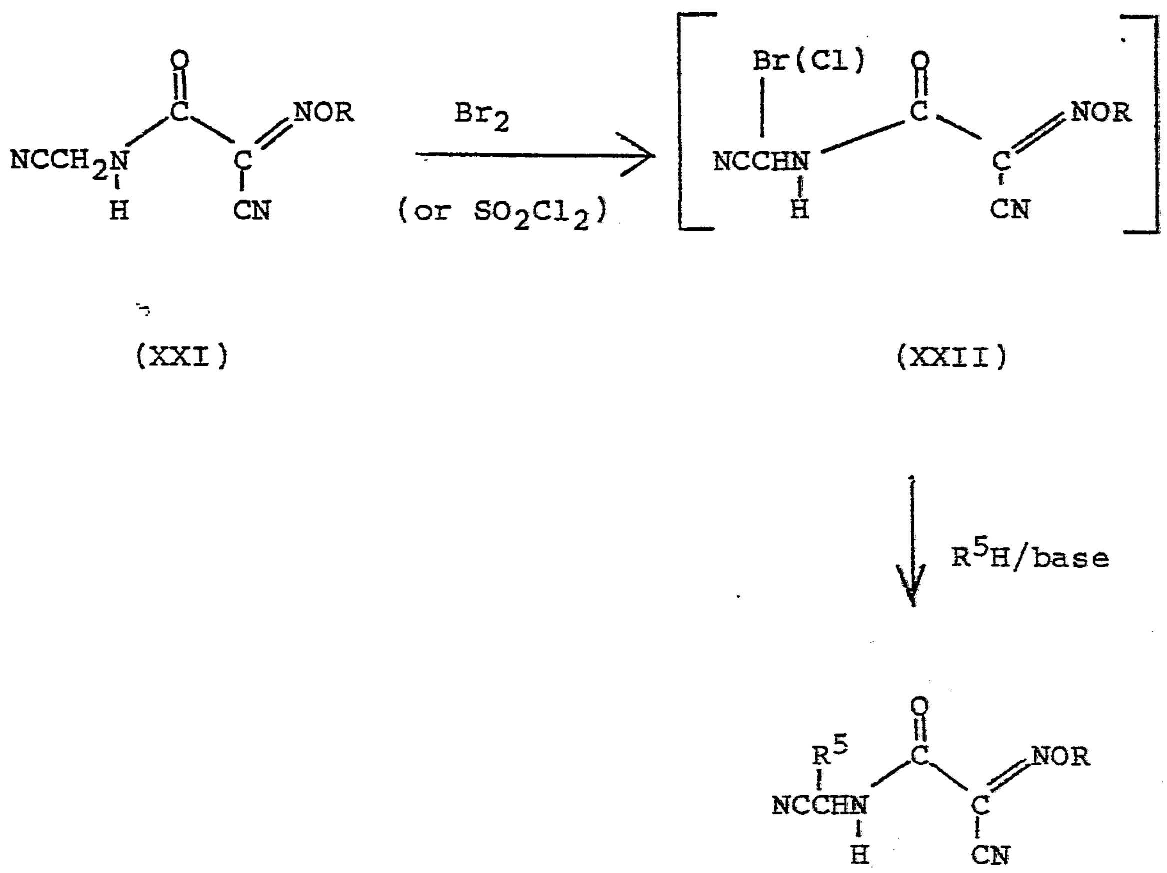


(XXI)

(XXII)

(XX)

In the second step of the reaction, the solution containing the reactive bromo- or chloro-nitrile (XXII), is treated directly with two or three equivalents of $R^5H$ when $R^5H$ is a 5-membered heterocyclic ring containing two to four nitrogen atoms, or (XXII) is treated with a large excess of $R^5H$, when $R^5$ is an alkoxy, alkenyloxy or alkynyloxy group as defined above, followed by an acid acceptor such as a tertiary amine (eg. triethylamine) or metal carbonate (eg. potassium carbonate), to give the

product (XX).

Route J

In route J a compound of formula (XXIV) where R is as defined in formula (I), is prepared by reaction of a compound of formula (XXIII) with formaldehyde (either paraformaldehyde or aqueous formaldehyde), a copper I salt such as cuprous bromide or iodide), and a secondary amine (particularly diisopropylamine) in a suitable solvent (such as dimethoxyethane, tetrahydrofuran or toluene) at 20°-150° (but preferably at 50-100°), and preferably under an inert atmosphere (such as nitrogen).

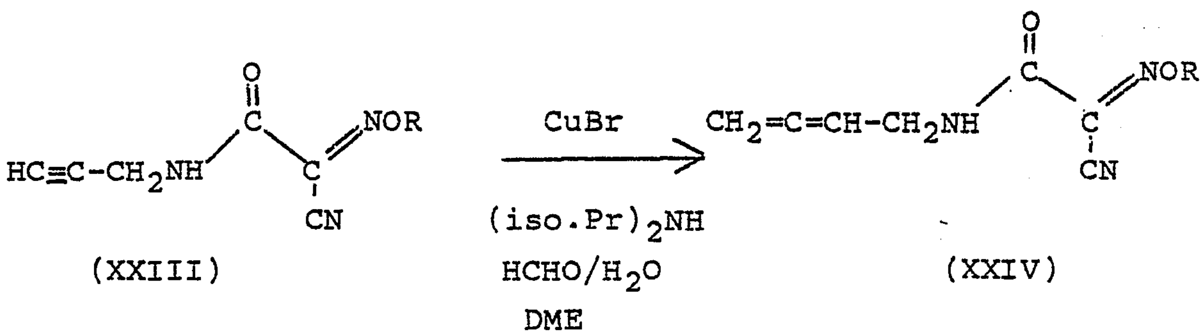


(XXIII) (XXIV)

Route K

In route K a compound of formula (XXVI) is prepared by treatment of a compound of formula (XXV) with aqueous sodium hydroxide to form a solution of the sodium salt, followed by a metal halide (such as cupric chloride, or ferric chloride) in water. The salt is isolated by filtration of the precipitate, or by evaporation of most of the water, treatment of the residue with ethanol, and filtration of the precipitated solid.

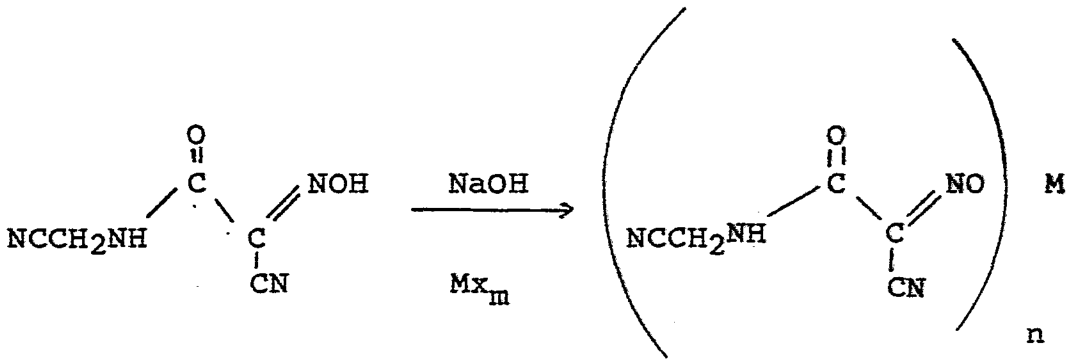


(XXV) (XXVI)

m = 1-3 n = 1-3

The present invention includes novel intermediates of formula (I) to (XXVI).

The compounds of formula I, and compositions containing them, are variously active against a wide range of fungal diseases, particularly, for example, against:

Plasmopara viticola (downy mildew) on vines and Phytophthora infestans (late blight) on potatoes and tomatoes and other species of Phytophthora.

Other downy mildews, powdery mildews and other fungal and bacterial diseases, for example:

Venturia inaequalis (scab) on apples
Pyricularia oryzae on rice
Cercospora arachidicola on peanuts and other Cercospora species
Xanthomonas oryzae on rice.

Some of the compounds have also shown a broad range of activities against fungi in vitro. They have activity against various pathogens which cause post-harvest diseases of fruit (eg. Penicillium digatatum and italicum on oranges and Gloeosporium musarum on bananas).

The compounds can move acropetally in the plant tissue.

The compounds may be used as such for fungicidal purposes but are more conveniently formulated into compositions for such usage. The invention thus provides a fungicidal composition comprising a compound of general formula (I) as hereinbefore defined, and, optionally, a carrier or diluent.

The invention also provides a method of combating fungi, which comprises applying to a plant, to seed of a plant, or to the locus of the plant or seed, a compound as hereinbefore defined, or a composition containing the same.

The compounds, can be applied in a number of ways, for example they can be applied, formulated or unformulated, directly to the foliage of a plant, or they can be applied also to bushes and trees, to seeds or to other medium in which plants, bushes or trees are growing or are to be planted, or they can be sprayed on, dusted on or applied as a cream or paste formulation, or they can be applied as a vapour; or as slow release granules. Application can be to any part of the plant, bush or tree, for example to the foliage, stems, branches or roots, or to soil surrounding the roots, or to the seed before it is planted; or to the soil generally, to paddy water or to hydroponic culture systems. The invention compounds may also be injected into plants or trees and they may also be sprayed onto vegetation using electrodynamic spraying techniques.

The term "plant" as used herein includes seedlings, bushes and trees. Furthermore, the fungicidal method of the invention includes preventative, protectant, prophylactic and eradicant treatment.

The compounds are preferably used for agricultural and horticultural purposes in the form of a composition. The type of composition used in any instance will depend upon the particular purpose envisaged.

The compositions may be in the form of dusting powders or granules comprising the active ingredient and a solid diluent or carrier, for example fillers such as kaolin, bentonite, kieselguhr, dolomite, calcium carbonate, talc, powdered magnesia, Fuller's earth, gypsum, Hewitt's earth, diatomaceous earth and China clay. Such granules can be preformed granules suitable for application to the soil without further treatment. These granules can be made either by impregnating pellets of filler with the active ingredient or by pelleting a mixture of the active ingredient and powdered filler. Compositions for dressing seed, for example, may comprise an agent (for example a mineral oil) for assisting the adhesion of the composition to the seed; alternatively the active ingredient can be formulated for seed dressing purposes using an organic solvent (for example N-methylpyrrolidone or dimethylformamide). The compositions may also be in the form of dispersible powders, granules or grains comprising a wetting agent to facilitate the dispersion in liquids of the powder or grains which may contain also fillers and suspending agents.

The aqueous dispersions or emulsions may be prepared by dissolving the active ingredient(s) in an organic solvent optionally containing wetting, dispersing or emulsifying agent(s) and then adding the mixture to water which may also contain wetting, dispersing or emulsifying agent(s). Suitable organic solvents are ethylene dichloride, isopropyl alcohol, propylene glycol, diacetone alcohol, toluene, kerosene, methylnaphthalene, the xylenes, trichloroethylene, furfuryl alcohol, tetrahydrofurfuryl alcohol, and glycol ethers (eg. 2-ethoxyethanol and 2-butoxyethanol).

The compositions to be used as sprays may also be in the form of aerosols wherein the formulation is held in a container under pressure in the presence of a propellant, eg. fluorotrichloromethane or dichlorodifluoromethane.

The compounds can be mixed in the dry state with a pyrotechnic mixture to form a composition suitable for generating in enclosed spaces a smoke containing the compounds.

Alternatively, the compounds may be used in a micro-encapsulated form. They may also be formulated in biodegradable polymeric formulations to obtain a slow, controlled release of the active substance.

By including suitable additives, for example additives for improving the distribution, adhesive power and resistance to rain on treated surfaces, the different compositions can be better adapted for various utilities.

The compounds can be used as mixtures with fertilisers (eg. nitrogen-, potassium- or phosphorus-containing fertilisers). Compositions comprising only granules of fertiliser incorporating, for example coated with, the compound are preferred. Such granules suitably contain up to 25% by weight of the compound. The invention therefore also provides a fertiliser composition comprising the compound of general formula (I) or a salt or metal complex thereof.

The compositions may also be in the form of liquid preparations for use as dips or sprays which are generally aqueous dispersions or emulsions containing the active ingredient in the presence of one or more surfactants eg. wetting agent(s), dispersing agent(s), emulsifying agent(s) or suspending agent(s); or which are spray formulations of the kind suitable for use in electrodynamic spraying techniques. The foregoing agents can be cationic, anionic or non-ionic agents. Suitable cationic agents are quaternary ammonium compounds, for example cetyltrimethyl-ammonium bromide.

Suitable anionic agents are soaps, salts of aliphatic monoesters of sulphuric acid (for example sodium lauryl sulphate), and salts of sulphonated aromatic compounds (for example sodium dodecylbenzenesulphonate, sodium, calcium or

ammonium lignosulphonate, butylnaphthalene sulphonate, and a mixture of sodium diisopropyl- and triisopropylnaphthalene sulphonates).

Suitable non-ionic agents are the condensation products of ethylene oxide with fatty alcohols such as oleyl or cetyl alcohol, or with alkyl phenols such as octyl- or nonyl-phenol and octylcresol. Other non-ionic agents are the partial esters derived from long chain fatty acids and hexitol anhydrides, the condensation products of the said partial esters with ethylene oxide, and the lecithins. Suitable suspending agents are hydrophilic colloids (for example polyvinylpyrrolidone and sodium carboxymethylcellulose), and the vegetable gums (for example gum acacia and gum tragacanth).

The compositions for use as aqueous dispersions or emulsions are generally supplied in the form of a concentrate containing a high proportion of the active ingredient(s), and the concentrate is to be diluted with water before use. These concentrates often should be able to withstand storage for prolonged periods and after such storage be capable of dilution with water in order to form aqueous preparations which remain homogeneous for a sufficient time to enable them to be applied by conventional and electrodynamic spray equipment. The concentrates may conveniently contain up to 95%, suitably 10-85%, for example 25-60%, by weight of the active ingredient(s). These concentrates suitably contain organic acids (eg. alkaryl or aryl sulphonic acids such as xylenesulphonic acid or dodecyl benzenesulphonic acid) since the presence of such acids can increase the solubility of the active ingredient(s) in the polar solvents often used in the concentrates. The concentrates suitably contain also a high proportion of surfactants so that sufficiently stable emulsions in water can be obtained. After dilution to form aqueous preparations,

such preparations may contain varying amounts of the active ingredient(s) depending upon the intended purpose, but an aqueous preparation containing 0.0005% or 0.01% to 10% by weight of active ingredient(s) may be used.

The compositions of this invention can comprise also other compound(s) having biological activity, eg. compounds having similar or complementary fungicidal activity or which possess plant growth regulating, or insecticidal activity.

The other fungicidal compound can be, for example, one which is capable of combating ear diseases of cereals (eg. wheat) such as Septoria, Gibberella and Helminthosporium spp., seed and soil borne diseases and downy and powdery mildews on grapes and powdery mildew and scab on apple etc. These mixtures of fungicides can have a broader spectrum of activity than the compound of general formula (I) alone; further the other fungicide can have a synergistic effect on the fungicidal activity of the compound of general formula (I). Examples of the other fungicidal compound are imazalil, benomyl, carbendazim, thiophanate-methyl, captafol, captan, sulphur, triforine, dodemorph, tridemorph, pyrazophos, furalaxyl, ethirimol, tecnazene, dimethirimol, bupirimate, chlorothalonil, vinclozolin, procymidone, iprodione, metalaxyl, fosetyl-aluminium, carboxin, oxycarboxin, fenarimol, nuarimol, fenfuram, methfuroxam, nitrotal-isopropyl, triadimefon, flutriafol, thiabendazole, etridiazole, triadimenol, biloxazol, dithianon, binapicryl, quinomethionate, guazatine, dodine, fentin acetate, fentin hydroxide, dinocap, folpet, dichlofluanid, ditalimphos, kitazin, cycloheximide, diclobutrazol, a dithiocarbamate, a copper compound, a mercury compound, 1-(2-cyano-2-methoxyiminoacetyl)-3-ethyl urea, fenapanil, ofurace, propiconazole, etaconazole and fenpropemorph, fenpropidine, topas, oxadixyl and prochloraz.

The compounds of general formula (I) can be mixed with soil, peat or other rooting media for the protection of plants against seed-borne, soil-borne or foliar fungal diseases.

Suitable insecticides are Pirimor, Croneton, dimethoate, "Metasystox" and formothion. The word "METASYSTOX" is a Trade Mark.

Suitable plant growth regulating compounds can be ones which control weeds or seedhead formation or selectively control the growth of the less desirable plants (eg. grasses). Some of these other agents will be herbicides.

Examples of suitable plant growth regulating compounds for use with the invention compounds are the gibberellins (eg. $GA_3$, $GA_4$ or $GA_7$), the auxins (eg. indoleacetic acid, indolebutyric acid, naphthoxyacetic acid or naphthylacetic acid), the cytokinins (eg. kinetin, diphenylurea, benzimidazole, benzyladenine or benzylaminopurine), phenoxyacetic acids (eg. 2,4-D or MCPA), substituted benzoic acid (eg. triiodobenzoic acid), morphactins (eg. chlorfluoroecol), maleic hydrazide, glyphosate, glyphosine, long chain fatty alcohols and acids, dikegulac, fluoridamid, mefluidide, substituted quaternary ammonium and phosphonium compounds (eg. chloromequat* chlorphonium or mepiquatchloride), ethephon, carbetamide, methyl-3,6- dichloroanisate, daminozide*, asulam, abscisic acid, isopyrimol, paclobutrazol, 1-(4-chlorophenyl)-4,6-dimethyl-2-oxo-1,2-dihydropyridine-3-carboxylic acid, hydroxybenzonitriles (eg. bromoxynil), difenzoquat, benzoylprop-ethyl 3,6-dichloropicolinic acid, and tecnazene.

The following Examples illustrate the invention; the temperatures are given in degrees Centrigrade (°C).

## EXAMPLE 1

This Example illustrates the preparation of compound No. 1 of Table I, by route A.

Step 1

The preparation of ethyl-2-cyano-2-methoxyiminoacetate having the structure

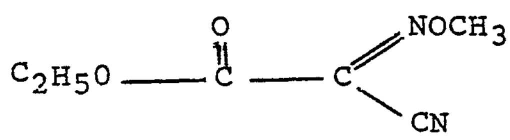

Ethyl 2-cyano-2-oximinoacetate (20.0g) was stirred in acetonitrile (150 ml) at room temperature and triethylamine (25 ml) was added. The methyl iodide (25 ml) was slowly added, whereupon a moderate exotherm occurred; and the solution went dark red. After completion of the addition and cooling, it was stirred for 24 hours. Excess solvent and methyl iodide were evaporated, and water added. The mixture was extracted with methylene chloride, which was dried over magnesium sulphate and evaporated to yield a dark oil (23.0 gm). This was purified by flash chromatography on Merck 9385 Kieselgel eluting with methylene chloride to give the pure product as a pale yellow oil (12.5 gm). Alternatively, the product was distilled at 105°-110°/10 mm, or was crystallised at -45°C from ethanol to give the product as a solid mp. 29-31°.

IR (liquid film) $\nu$ ; 1755, 1730 $cm^{-1}$.

NMR ($CDCl_3$) $\delta$ ; 1.42 (t,3H); 4.30 (s,3H); 4.40 (q,2H).

<u>Step 2</u>

The preparation of the compound <u>2-cyano-2-methoxyimino-N-propargylacetamide</u> having the structure :

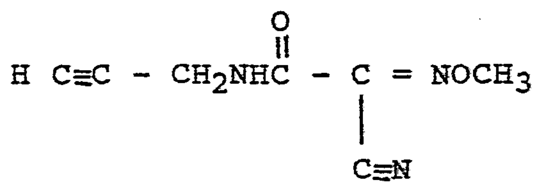

(compound No. 1 of Table I).

Propargylamine hydrochloride (1.59 gm), ethyl 2-cyano-2-methoxyiminoacetate (2.80 gm) and triethylamine (1.80 gm) were stirred in ethanol (100 ml) at room temperature for 6 hours and stood overnight. The ethanol was evaporated and the residue triturated with water. The tan solid was filtered and was dried to give the product (3.85 gm), mpt 84-86°C.

IR (nujol) $\nu$ ; 3370, 3300, 2245, 2130, 1695 ($cm^{-1}$)

NMR ($CDCl_3$) $\delta$ ; 3.04 (t,1H); 3.90 (m,2H); 4.16 (s,3H); 8.95 (bt,1H)

EXAMPLE 2

This Example illustrates the preparation of <u>2-cyano-2-methoxyimino-N-2,3-diiodo-allylacetamide</u> (compound 7 of Table I), having the structure

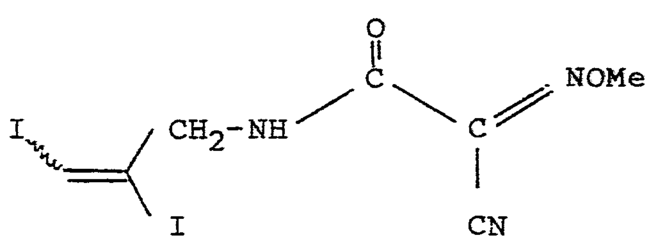

Iodine (0.245 gm) was added as a slurry in chloroform (5 ml) to the propargyl amide from the previous reaction (0.275 gm) in chloroform (25 ml) at room temperature. The mixture was stirred for 7 hours, was then stood overnight and then for eight weeks. After that time the solution had decolourised.

The solvent was evaporated to yield a viscous oil which yielded a whitish solid on trituration with ether. This was recrystallised from petroleum (40:60)/toluene to give material mpt 96-8°C (0.210 gm). Then NMR spectrum showed that this was a mixture of E and Z double bond isomers.

IR (nujol) $\nu$ ; 3330, 1680 $cm^{-1}$

NMR ($CDCl_3$) $\delta$ ; 4.30 (s,4H); 4.38 (s,1H); 6.70 (bs,1H); 7.20 (s,1H)

EXAMPLE 3

This Example illustrates the preparation of compound 9 of Table I by route B.

<u>Stage 1</u>

The preparation of the intermediate <u>sodium 2-cyano-2-methoxyiminoacetate</u> having the structure

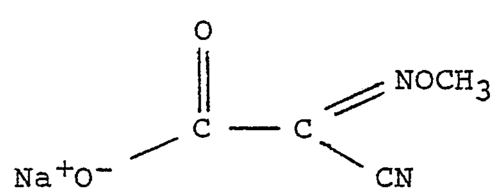

Ethyl 2-cyano-2-methoxyiminoacetate (7.2 gm) was added to a solution of powdered sodium hydroxide (1.8 gm) in ethanol (40 ml). The mixture was stirred for 40 minutes at room temperature and then the ethanol evaporated at 30°C, giving the sodium salt as a white solid (6.07 gm), which was dried by pumping at 0.1 mm and storing in a desiccator over phosphorus pentoxide.

NMR ($D_2O$) $\delta$ ; 4.20 (s)

Stage 2

The preparation of 2-cyano-2-methoxyiminoacetyl chloride having the structure

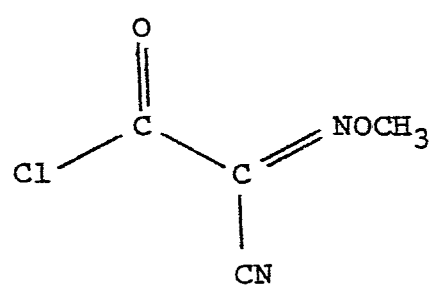

The dry sodium salt (1.50 gm) was stirred as a suspension in dry toluene (20 ml) at room temperature. Oxalyl chloride (1.50 gm) in dry toluene (1.5 ml) was added in portions over 10 minutes followed by one drop of dimethylformamide. Vigorous evolution of gas took place over about 0.5 hours. After stirring for a further one hour, the suspension was filtered through celite and then evaporated to remove toluene, excess oxalyl chloride and hydrogen chloride gas. The reactive acid chloride was used without further purification (1.5 gm), although it can be distilled at 83-84°/20 mm to give a pale yellow oil.

Stage 3

The preparation of the compound 2-cyano-2-methoxyimino-N,N-dipropargyl-acetamide having the structure

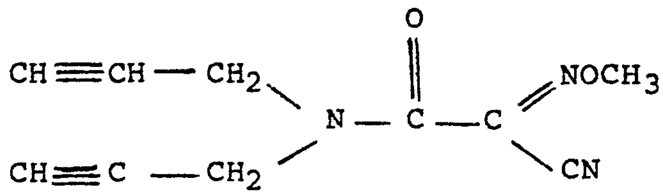

(Compound 9 of Table I)

2-Cyano-2-methoxyiminoacetyl chloride (0.380 gm) in dry ether (5 ml) was pipetted into a stirred solution of dipropargylamine (0.254 gm) in dry toluene (20 ml), containing triethylamine (0.270 gm), at room temperature. After the immediate precipitation of a white solid the mixture was stirred for 0.5 hours and then stood overnight.

It was then diluted with ethyl acetate and poured into water. The ethyl acetate layer was washed with aqueous sodium bicarbonate and water, and the dried over magnesium sulphate. Evaporation then yielded a viscous oil. This was purified by preparative thin layer chromatography eluting with methylene chloride, to give the product as a viscous oil (0.185 gm).

IR (liquid film) $\nu$ ; 3280, 2120 (W), 1655 $cm^{-1}$

NMR ($CDCl_3$) $\delta$ ; 2.32 (s,2H); 4.22 (s,3H); 4.40 (s,4H)

EXAMPLE 4

This Example illustrates the preparation of methoxycarbonylmethyl-(2-cyano-2-methoxyimino)-acetamide (compound 24 of Table I) by route B, having the structure

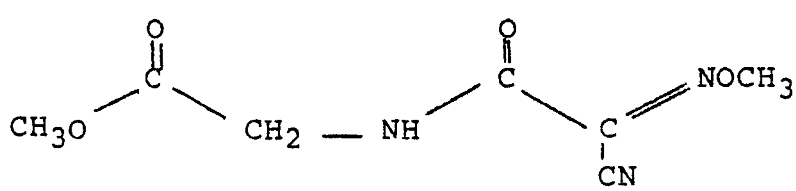

2-Cyano-2-methoxyiminoacetyl chloride (1.0 gm) in dry toluene (10 ml) was added over 5 minutes to a vigorously stirred mixture of glycine methyl ester hydrochloride (0.860 gm) in water (5 ml) containing sodium bicarbonate (1.2 gm) at room temperature. The mixture was stirred vigorously for four hours and then diluted with water. The aqueous layer was extracted with ethyl acetate (4 x 50 ml) and the ethyl acetate layer dried over magnesium sulphate.

Evaporation of the solvent then yielded a vigorous oil which slowly crystallised. Trituration with ether gave an off-white solid mpt. 65-67° (0.825 gm).

IR (nujol) $\nu$ ; 3330, 1680 $cm^{-1}$

NMR ($CDCl_3$) $\delta$ ; 3.80 (s,3H); 4.16 (d,2H); 4.28 (s,3H); 7.05 (bs,1H)

EXAMPLE 5

This Example illustrates the preparation of 2-cyano-2-methoxyimino-N-(propargyl)-N-allylacetamide (Compound 10 of Table I), by route C, having the structure

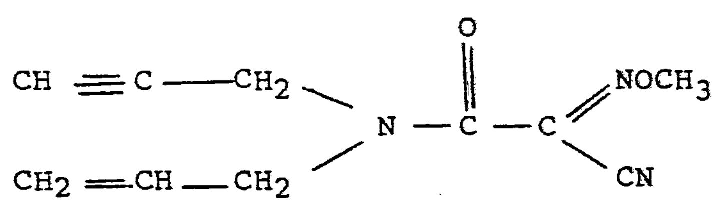

2-Cyano-2-methoxyimino-N-propargylacetamide (0.33 gm) was stirred in dry DMF (20 ml) with allyl bromide (0.30 gm) at room temperature. Sodium hydride (0.10 gm of a 50% dispersion in oil) was added in portions over 30 minutes, and the mixture stirred for two hours after completion of the addition. The reaction was then poured into water, extracted with ethyl acetate and the ethyl acetate fraction dried over magnesium sulphate and evaporated to give an oil (0.80 gm). This was purified by medium pressure liquid

chromatography eluting with ethyl acetate/40:60 petroleum ether (1:3) to give the pure product as a clear pale yellow oil (0.22 gm).

IR (liquid film) $\lambda$ ; 3300, 2230 (W), 2120 (W), 1655 $cm^{-1}$

NMR ($CDCl_3$) $\delta$ ; 2.30 (m,1H); 4.10-4.30 (m,7H); 5.10-5.40 (m,2H); 5.60-6.00 (m,1H)

EXAMPLE 6

This Example illustrates the preparation of 2-cyano-2-methoxyimino-N-2-thiazolylacetamide (Compound 25 of Table I), by route D, having the structure

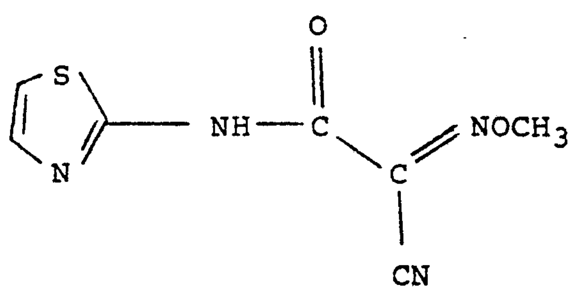

Sodium hydride (0.25 gm of a 50% dispersion in oil) was added in portions over 10 minutes to 2-aminothiazole (0.50 gm) stirred in dry dimethoxyethane (DME, 15 ml) at room temperature. After completion of the vigorous effervescence a cream suspension was formed. Ethyl 2-cyano-2-methoxy-iminoacetate (0.75 gm) in dry DME (2 ml) was added all at once. The solution went very dark and was then stirred for 1.5 hours. The solution was poured carefully into water and then extracted with ethyl acetate. The aqueous layer was then acidified with dilute hydrochloric acid and extracted with ethyl acetate. This extract was dried over magnesium sulphate and evaporated to yield an orange solid. This was triturated with methanol to give the pure product (0.30 gm) mpt. 203-205°C.

IR (nujol) $\nu$ ; 3200, 1610 $cm^{-1}$

NMR (DMSO-$d^6$) $\delta$ ; 4.20 (s,3H); 7.20 (d,1H); 7.46 (d,1H);

EXAMPLE 7

This Example illustrates the preparation of 2-cyano-2-methoxyimino-N-(cyanomethyl)-acetamide (Compound No. 20 of Table I), by route B, having the structure :

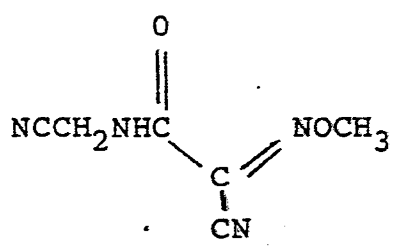

2-Cyano-2-methoxyimino acetyl chloride (0.75 gm) in dry toluene (10 ml) was addded all at once to a vigorously stirred solution of aminoacetonitrile bisulphate (0.77 gm) in water (5 ml) containing sodium bicarbonate (1.20 gm). The two-phase mixture was stirred rapidly at room temperature for four hours and stood overnight, and then extracted three times with ethyl acetate. The ethyl acetate fractions were combined, dried over magnesium sulphate and evaporated to give a viscous oil. After trituration with diethylether/petroleum ether (40:60) a white solid was obtained (0.172 gm), mpt. 83-85°.

IR (nujol) $\nu$ ; 3360, 1675 $cm^{-1}$

NMR (acetone-$d^6$) $\delta$ ; 4.40 (s,3H); 4.51 (s,2H), 8.68 (s,1H,NH)

## EXAMPLE 8

This Example illustrates the preparation of 2-cyano-2-methoxyimino-N-( α-cyanoethyl)-acetamide (Compound No. 34 of Table 1), by Route B, having the structure :

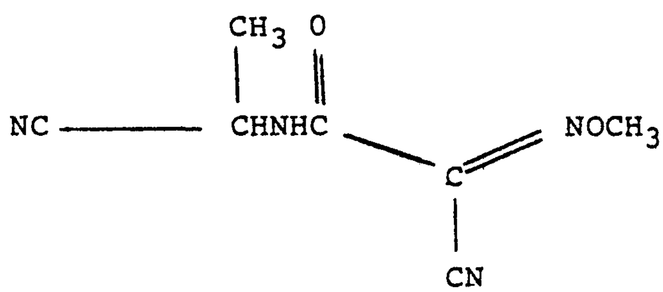

A mixture of α-cyanoethylamine (0.70g) and triethylamine (1.01g) in ethyl acetate (5ml) was added slowly dropwise to 2-cyano-2-methoxyiminoacetylchloride (1.47g) in ethyl acetate (20ml) at 15°C, keeping the temperature controlled to less than 20°C during the addition. The mixture became orange and then a white precipitate appeared; after completion of the addition the mixture was stood overnight. The reaction was then poured into water and the ethyl acetate layer washed with saturated sodium bicarbonate, and then brine, before drying over magnesium sulphate. The ethyl acetate was evaporated to give a viscous oil. This was purified twice by high pressure liquid chromatography, on Merck Kiesel gel 9385 eluting with 1:1 ethyl acetate: 40/60 petroleum ether, to give the desired product as an oil (0.419g).

IR (liquid film) $\nu$ ; 3330 (s), 2240 (w), 1675 (s) $cm^{-1}$

NMR (DMSO-$d^6$) $\delta$ ; 1.38 (d,3H), 4.18 (s,3H), 4.90 (q, 1H), 9.40 (bs,NH)

EXAMPLE 9

This Example illustrates the preparation of 2-cyano-2-methoxyimino-N-[(2-furyl)-α-cyano-methyl]-acetamide (Compound No. 63 of Table 1), by Route B, having the structure :

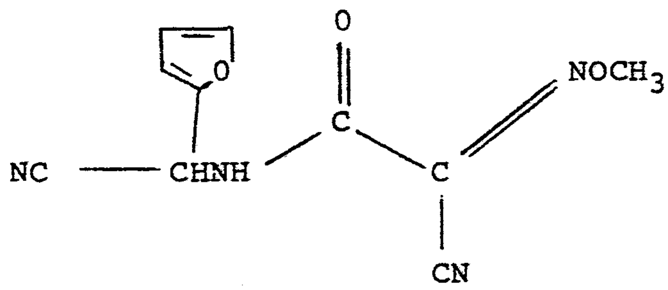

α-Cyano-2-furylmethylamine hydrochloride (1.98g) was dissolved in water (20ml) and filtered to remove insoluble solid. The solution was then treated with aqueous sodium hydroxide (5.5ml 2M solution) until pH7 was reached, followed by sodium bicarbonate (0.84g) and ethyl acetate (20ml). To this mixture was added 2-cyano-2-methoxyiminoacetyl chloride (1.47g) in ethyl acetate (30ml), slowly dropwise whilst cooling the reaction to maintain the temperature to below 20°C. After completion of the addition (40 minutes) the reaction was stirred for 1.5 hours and then poured into water. The aqueous fraction was extracted with further ethyl acetate, the extracts combined, dried over magnesium sulphate and evaporated to yield an oil (2.4g). The oil was purified by flash chromatography on silica gel, eluting with methylene chloride to give the product as a golden orange oil (1.94g).

IR (liquid film) λ ; 3303 (s), 2230 (w), 1680 (s) $cm^{-1}$

NMR ($CDCl_3$) δ ; 4.32 (s,3H), 6.22 (d,1H), 6.45 (m,1H), 6.62 (m,1H), 7.32 (bd,1H), 7.50 (m,1H)

EXAMPLE 10

This Example illustrates the preparation of Compound No. 31 of Table 1, by Route E.

Step 1

The preparation of N-(cyanomethyl)-cyanoacetamide having the structure :

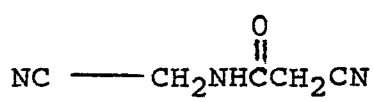

Dry cyanoacetic acid (2.98g) was dissolved in dry acetonitrile (35ml) and dry aminoacetonitrile (1.96g, obtained by neutralising aminoacetonitrile hydrogen sulphate in water with sodium hydroxide, extraction with ethylacetate and evaporation) was added to the colourless solution, and the mixture cooled to 5°C. 4-Dimethylaminopyridine (catalytic amount) was then added followed by dicyclohexylcarbodiimide (7.94g) portionwise. After 1 hour stirring at 5°C the reaction was stood overnight. Glacial acetic acid (0.7ml) was then added to the reaction mixture and after 1 hour a solid filtered (dicyclohexylurea) and the filtrate evaporated to give a red oil which crystallised on trituration with diethylether (6.67g). The solid was recrystallised from ethanol to give the desired product as light brown leaflets (2.65g) mpt:94-96°C.

IR (nujol) $\nu$ ; 3260(s), 2260(w), 1660(s), $cm^{-1}$

NMR (DMSO-$D^6$) $\delta$ ; 3.70 (s,2H), 4.14 (d,2H), 8.83 (s,NH)

Step 2

The preparation of 2-cyano-2-oximino-N-(cyanomethyl)-acetamide having the structure :

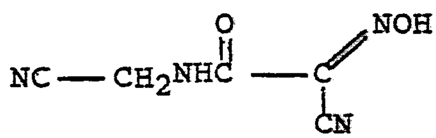

N-(cyanomethyl)-cyanoacetamide (6.16g) was stirred in water (140ml) at room temperature, and sodium nitrite (6.90g) was added to the solution followed by glacial acetic acid (6.90g). After stirring at room temperature for 15 minutes, the reaction mixture was warmed to 45-50°C (effervescence) for 3 hours, and then cooled to room temperature. The dark green solution was extracted with ethyl acetate, and the organic fraction was then washed with aqueous sodium bicarbonate. The aqueous layers were acidified to pH4 with concentrated hydrochloric acid, and then extracted with ethyl acetate, and the ethyl acetate layers dried over magnesium sulphate, and evaporated to give a pale yellowish solid (3.2g) mpt 144-146°C.

IR (nujol) $\nu$ ; 3320 (s), 3100 (s), 1650 (s) $cm^{-1}$

NMR (DMSO-$d^6$)$\delta$ ; 4.16 (d,2H), 9.06 (t,NH), 14.72 (bs,OH)

EXAMPLE 11

This Example illustrates the preparation of 2-cyano-2-ethoxyimino-N-(cyanomethyl)-acetamide (Compound No. 22 from Table 1), by Route E, having the structure :

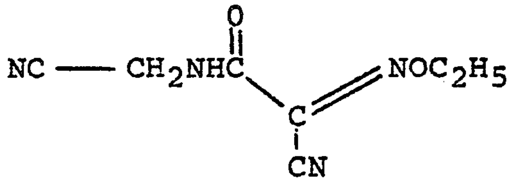

2-Cyano-2-oximino-N-(cyanomethyl)-acetamide (0.46g) was stirred in acetone (15ml) and potassium carbonate added (0.21g), the reaction mixture was cooled to 15°C and diethylsulphate (0.46g) in acetone (2mls) was added dropwise. The reaction was stirred at 15°C for 15 minutes, warmed to room temperature and after 1 hour the solvent evaporated. The residue was partitioned between ethyl acetate and water, the aqueous layer extracted again and the organic fractions dried over magnesium sulphate and evaporated to give an oil which crystallised (0.52g). This solid was purified by high pressure liquid chromatography on Merck Kieselgel 9385, eluting with ethyl acetate to give the pure product as a crystalline solid (0.37g) mpt 73-75°C.

IR (nujol $\lambda$) ; 3340 (s), 2240 (w), 1670 (m) $cm^{-1}$

NMR ($CDCl_3$) $\delta$ ; 1.40 (t,3H), 4.25 (d,2H), 4.52 (q,2H), 7.05 (bs,NH)

EXAMPLE 12

This Example illustrates the preparation of Compound No 54 of Table I, by Route H.

Step 1

The preparation of <u>benzyloxycarbonylaminoacetamide</u> having the structure :

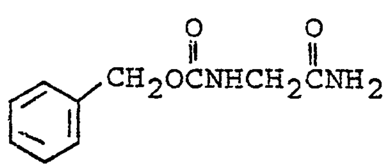

Benzylchloroformate (22.4ml) was added dropwise with vigorous stirring to glycinamide hydrochloride (16.58g) in water (50ml) containing sodium hydrogen carbonate (30.0g), over 5 minutes. The reaction mixture was stirred at room temperature for 65 hours, and the white solid filtered, washed with water, air dried and recrystallised from ethyl acetate/:60/80 petroleum ether to give the white crystalline product (15.96g).

NMR (DMSO-$d^6$) $\delta$ ; 3.58 (d,2H), 5.05 (s,2H), 7.00 (bs,1H), 7.20-7.48 (m,7H)

Step 2

The preparation of benzyloxycarbonylaminothioacetamide

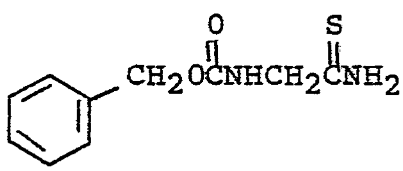

Benzyloxycarbonylaminoacetamide (2.08g) was dissolved in dioxan and phosphorus pentasulphide (1.10g) added and the whole heated to reflux with stirring for 45 minutes and then cooled to room temperature. The reaction mixture was poured into water, which was then extracted with ethyl acetate; the ethyl acetate fraction was then dried over magnesium sulphate and evaporated to give an oil which crystallised to a soft solid (2.91g). This was purified by high pressure liquid chromatography to give the desired product as a white solid (0.80g), mpt 140-142°C.

IR (nujol) $\nu$ ; 3380 (s), 3285 (s), 3215 (s), 1695 (s), $cm^{-1}$

NMR (DMSO-$d^6$) $\delta$ ; 3.90 (d,2H), 5.02 (s,2H)

Step 3

The preparation of aminothioacetamide hydrobromide having the structure :

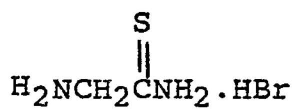

Benzyloxycarbonylaminothioacetamide (2.77g) was stirred in glacial acetic acid (10ml) and hydrogen bromide in acetic acid (12.4g of a 45% solution) was added dropwise to the slurry over a few minutes. The solid amide gradually dissolved until a clear yellow solution was obtained. After 10 minutes a yellow precipitate was formed. After stirring for a further 1 hour at room temperature, diethylether (100ml) was added, and after a further 15 minutes the solid was filtered and washed well with diethylether and then air dried to give the product as a hygroscopic pale yellow solid (2.22g), which was used directly for the next reaction without purification.

NMR (DMSO-$d^6$)$\delta$ ; 3.82 (s).

Step 4

The preparation of 2-cyano-2-methoxyimino-N-(thiocarbamoylmethyl)-acetamide having the structure :

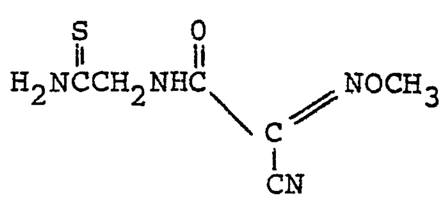

Aminothioacetamide hydrobromide (1.71g) was stirred in water (25ml) and sodium bicarbonate was added. The reaction mixture was stirred vigorously until all

effervescence ceased and then ethyl acetate added (40ml). After stirring for a further 5 minutes, 2-cyano-2-methoxyiminoacetyl chloride (1.61g) in ethyl acetate (40ml) was added dropwise. After completion of the addition the reaction was stirred for 1.5 hours and then stood overnight. The mixture was then poured into water, the organic layer washed with water several times and dried over magnesium sulphate and then evaporated to give a brown oil which crystallised. After trituration with ether a tan powder was obtained (0.69g). This was recrystallised from ethyl acetate to give the product as a light brown crystalline solid (0.53g) mpt 120-121°C.

IR (nujol ν ; 3400 (w), 3320 (s), 3175 (s), 2225 (w), 1665 (s) $cm^{-1}$.

NMR (DMSO-$d^6$) δ; 4.19 (s,3H), 4.50 (d,2H), 8.80 (t,NH), 9.08 (bs,NH), 2.70 (bs,NH).

EXAMPLE 13

This Example illustrates the preparation of Compound No 54 of Table 1, by Route H.

Step 1

The preparation of benzyloxycarbonylaminoacetonitrile having the structure :

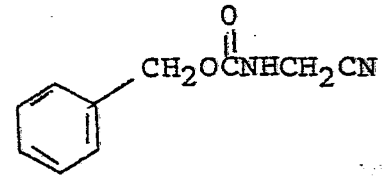

Aminoacetonitrile (5.6g) in water (30ml) was treated with sodium bicarbonate (9.24g), followed by dropwise addition of benzyl chloroformate (17.9g) with vigorous

stirring over 10 minutes. When addition was complete the reaction mixture was stirred at room temperature for 22 hours. The solid precipitated was filtered, washed with water, air dried and recrystallised from ethyl acetate:60/40 petroleum ether to give the white crystalline produce (13.35g) mp 60-62°C.

IR (Nujol) $\nu$ ; 3290, 1680 $cm^{-1}$.

NMR ($CDCl_3$) $\delta$ ; 4.00 (d,2H), 5.17 (s,2H), 5.64 (bs,NH), 7.40 (s,5H).

<u>Step 2</u>

The Example illustrates the preparation of <u>benzyloxycarbonylaminothioacetamide</u> by Route H having the structure :

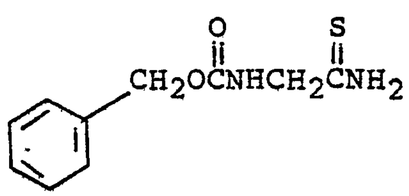

Hydrogen sulphide gas was bubbled through dry toluene (25ml) for a few minutes and then benzyloxycarbonylaminoacetonitrile (0.95g) added and stirred at room temperature until it had dissolved, and then triethylamine (0.51g) added. After a few minutes hydrogen sulphide gas was again bubbled through the reaction mixture, for 30 minutes, by which time a white precipitate had appeared from the yellow solution, and the mixture was then stood overnight. The solid was then filtered, washed with toluene and diethyl ether to give the product as a white powdery solid (0.92g) mpt 140-142°C identical to material prepared by Route G.

EXAMPLE 14

This Example illustrates the preparation of 2-cyano-2-methoxyimino-N-[ethoxy-(α-cyano)-methyl]acetamide (Compound No. 97 from Table I), by route I, having the structure :

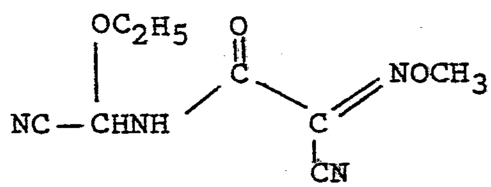

2-Cyano-2-methoxyimino-N-(cyanomethyl)-acetamide (0.83g, compound No. 20 from Table I), was stirred in ethyl acetate (25 ml), and cooled to 5°C. Two drops of a solution of bromine (0.80g) in ethyl acetate (5 ml) was added, and the reaction mixture warmed to room temperature for 1 hour, and then warmed to 30°C while more bromine solution was added. No decolourisation occurred. The mixture was then warmed to 50°C and the remaining bromine solution added; the mixture gradually became yellow and a cloudy precipitate appeared. The mixture was filtered and methanol (10 ml) was added to the filtrate, followed by triethylamine (1.01g) in ethyl acetate (5 ml) two minutes later. The mixture was then washed with water, dried over magnesium sulphate and evaporated to give an orange oil (0.7g), which was purified by high pressure liquid chromatography on Merck Kieselgel 9385, eluting with 40-60° petroleum/ethyl acetate, 2:1, to give the product as a pale yellow oil (0.46g).

IR (nujol) $\nu$ : 3310 (s), 1685 (s) $cm^{-1}$

NMR ($CDCl_3$) $\delta$ : 1.20 (t,3H); 3.66 (q,2H); 4.28 (s,3H); 6.00 (d,1H); 7.44 (bd,1H)

EXAMPLE 15

This Example illustrates the preparation of 2-cyano-2-methoxyimino-N-[1-pyrazolyl-(2-cyano)methyl]-acetamide (Compound No. 98 from Table I), by route I, having the structure :

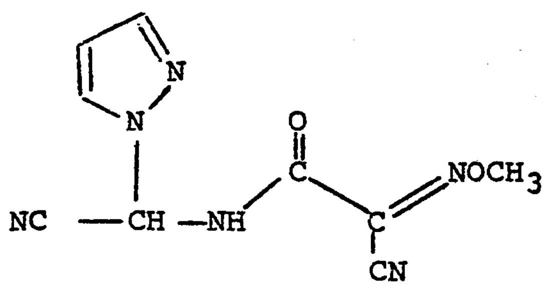

2-Cyano-2-methoxyimino-N-(cyanomethyl)acetamide (0.83g) in ethyl acetate (25 ml) was brominated in exactly the same manner as in Example 14, with bromine (0.80g) at 50°C. The resulting yellow solution was cooled to room temperature, stirred for 15 minutes, and then pyrazole (1.02g) in ethyl acetate (10 ml) was added in one portion. An immediate precipitation took place.

The mixture was then filtered, the filtrate was washed with water, dried over magnesium sulphate and then evaporated to give a pale yellow oil (1.17g), which crystallised. This was recrystallised from ethyl acetate/petroleum to give the product (0.69g) mp. 167° (dec.)

IR (nujol) ν : 3150 (s), 1675 (s) $cm^{-1}$

NMR (DMSO-$d^6$) δ : 4.20 (s,3H); 6.32 (t,1H); 7.40 (s,1H); 7.58 (d,1H); 7.86 (d,1H); 11.55 (bs,1H)

EXAMPLE 16

This Example illustrates the preparation of 2-cyano-2-methoxyimino-N-allenylmethylacetamide (Compound No. 107 from Table I) by route J, having the structure :

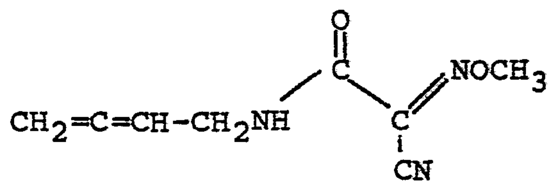

2-Cyano-2-methoxyimino-N-propargyl-acetamide (0.495g), diisopropylamine (0.33g), 37% aqueous formaldehyde (0.50g) and cuprous bromide (0.140g) were refluxed in dimethoxyethane (20 ml) under nitrogen for 8 hours. .The solution was cooled, filtered and the solvent evaporated. The oil residue was dissolved in ethyl acetate, washed with dilute aqueous acetic acid, dilute aqueous sodium bicarbonate and then brine, dried over magnesium sulphate and evaporated to give the product as an orange oil.

This was purified by flash chromatography on Merck Kieselgel 9385, eluting with ethyl acetate/petroleum, 1:1, to give pure product (0.175g).

IR (liquid film) $\nu$ : 3350 (m), 1960 (w), 1685 (s) $cm^{-1}$

NMR ($CDCl_3$) $\delta$ : 3.85-4.10 (m,2H); 4.27 (s,3H); 4.80-4.96 (m,2H); 5.21 (q,1H)

EXAMPLE 17

This Example illustrates the preparation of the Copper salt of 2-cyano-2-oximino-N-cyanomethyl-acetamide (Compound No. 43 of Table I) by route K.

2-Cyano-2-oximino-N-cyanomethylacetamide (1.02g) was stirred in water (5 ml) at room temperature, sodium hydroxide (3.5 ml of a 2N solution) was added. To the resultant brown solution was added copper $^{II}$ chloride (0.46g) in water (10 ml), giving a greenish-black solution. The mixture was then stood overnight. The bulk of the water was evaporated and ethanol (35 ml) added. A solid was obtained on scratching which was filtered, washed with ether and air dried to give the copper $^{II}$ salt as blue-green solid (0.939g), mp. $>$ 300°.

IR (nujol) $\delta$ : 3385 (s), 2240 (w) $cm^{-1}$

EXAMPLE 18

This Example illustrates the preparation of iron $^{III}$ salt of 2-cyano-2-oximino-N-cyanomethyl acetamide (Compound No. 45 of Table I), by route K.

To a mixture of 2-cyano-2-oximino-N-cyanomethyl-acetamide (1.0g) in water (5 ml) at room temperature was added sodium hydroxide (3.5 ml of 2N solution). To this brown solution was added ferric chloride (0.38g) in water (10 ml). A muddy brown solid precipitated. After 1 hour the solid was filtered and washed with ether, and air dried to give the ferric salt as a brown solid (0.453g), mp. $>$300°.

IR (nujol) $\delta$ : 3375 (b) $cm^{-1}$

EXAMPLE 19

An emulsifiable concentrate was made up by mixing the ingredients, and stirring the mixture until all the constituents were dissolved.

| | |
|---|---|
| Compound of Example 34 | 10% |
| Ethylene dichloride | 40% |
| Calcium dodecylbenzenesulphate | 5% |
| "Lubrol" L | 10% |
| "Aromasol" H | 35% |

The words "Lubrol" and "Aromasol" are Trade Marks.

EXAMPLE 20

A composition in the form of grains readily dispersible in a liquid, eg. water, was prepared by grinding together the first three ingredients in the presence of added water and then mixing in the sodium acetate. The resultant mixture was dried and passed through a British Standard mesh sieve, size 44-100, to obtain the desired size of grains.

| | |
|---|---|
| Compound of Example 36 | 50% |
| "Dispersol" T | 25% |
| "Lubrol" APN5 | 1.5% |
| Sodium acetate | 23.5% |

The words "Dispersol" and "Lubrol" are Trade Marks.

EXAMPLE 21

The ingredients were all ground together to produce a powder formulation readily dispersible in liquids.

| | |
|---|---|
| Compound of Example 54 | 45% |
| 'Dispersol" T | 5% |
| "Lissapol" NX | 0.5% |
| "Cellofas" B600 | 2% |
| Sodium acetate | 47.5% |

The words "Lissapol", "Dispersol" and "Cellofas" are Trade Marks.

## EXAMPLE 22

The active ingredient was dissolved in a solvent and the resultant liquid was sprayed on to the granules of China clay. The solvent was then allowed to evaporate to produce a granular composition.

| | |
|---|---|
| Compound of Example 20 | 5% |
| China clay granules | 95% |

## EXAMPLE 23

A composition suitable for use as a seed dressing was prepared by mixing the three ingredients.

| | |
|---|---|
| Compound of Example 22 | 50% |
| Mineral oil | 2% |
| China clay | 48% |

## EXAMPLE 24

A dusting powder was prepared by mixing the active ingredient with talc.

| | |
|---|---|
| Compound of Example 63 | 5% |
| Talc | 95% |

EXAMPLE 25

A col formulation was prepared by ball-milling the constituents set out below and then forming an aqueous suspension of the ground mixture with water.

| | |
|---|---|
| Compound of Example 85 | 40% |
| "Dispersol" T | 10% |
| "Lubrol" APN5 | 1% |
| Water | 49% |

The words "Dispersol" and "Lubrol" are Trade Marks.

EXAMPLE 26

A dispersible powder formulation was made by mixing together the ingredients set out below and then grinding the mixture until all were thoroughly mixed.

| | |
|---|---|
| Compound of Example 27 | 25% |
| "Aerosol" OT/B | 2% |
| "Dispersol" A.C. | 5% |
| China clay | 28% |
| Silica | 40% |

The words "Aerosol" and "Dispersol" are Trade Marks.

EXAMPLE 27

This Example illustrates the preparation of a dispersible powder formulation. The ingredients were mixed and the mixture then ground in a communition mill.

| | |
|---|---|
| Compound of Example 37 | 25% |
| "Perminal" BX | 1% |
| "Dispersol" T | 5% |

| | |
|---|---|
| Polyvinylpyrrolidone | 10% |
| Silica | 25% |
| China clay | 34% |

The words "Perminal" and "Dispersol" are Trade Marks.

EXAMPLE 28

The ingredients set out below were formulated into a dispersible powder by mixing then grinding the ingredients.

| | |
|---|---|
| Compound of Example 68 | 25% |
| "Aerosol" OT/B | 2% |
| "Dispersol" A | 5% |
| China clay | 68% |

The words "Aerosol" and "Dispersol" are Trade Marks.

In Examples 19 to 28 the proportions of the ingredients given are by weight. The remaining compounds of Table I were all similarly formulated as per Examples 19 to 28.

There now follows an explanation of the compositions or substances represented by the various Trade Marks mentioned above.

| | |
|---|---|
| "LUBROL" L : | a condensate of nonyl phenol (1 mole) with ethylene oxide (13 moles) |
| "AROMASOL" H : | a solvent mixture of alkylbenzene |
| "DISPERSOL" T & AC : | a mixture of sodium sulphate and condensate of formaldehyde with sodium naphthalene sulphonate |

| | |
|---|---|
| "LUBROL" APN5 : | a condensate of nonyl phenol (1 mole) with naphthalene oxide (5.5 moles) |
| "CELLOFAS" B600 : | a sodium carboxymethyl cellulose thickener |
| "LISSAPOL" NX : | a condensate of nonyl phenol (1 mole) with ethylene oxide (8 moles) |
| "AEROSOL" OT/B : | dioctyl sodium sulphosuccinate |
| "PERMINAL" BX : | a sodium alkyl naphthalene sulphonate |

EXAMPLE 29

The compounds were tested against a variety of mainly foliar fungal diseases of plants. The techniques employed were as follows.

For all tests the plants were grown in John Innes Potting Compost (No. 1 or 2) in 4 cm diameter minipots. The test compounds were formulated either by bead milling with aqueous Dispersol T or as a solution in acetone or acetone/ethanol which was diluted to the required concentration immediately before use. The solutions or suspensions (100 ppm ia.) were sprayed on the foliage and applied to the roots of the plant via the soil. The sprays were applied to maximum retention and the root drenches to a final concentration equivalent to approximately 40 ppm ai./dry soil. "TWEEN" 20, to give a final concentration of 0.05%, was added when the sprays were applied to cereals. (a.i. means "active ingredient"). The word "TWEEN" is a Trade Mark.

Most were protectant tests where the compound was applied to the soil and roots and to the foliage one or two days before the plant was inoculated with the pathogen. The foliar pathogens were applied by spraying as spore suspensions onto the leaves of the test plants.

After inoculation, the plants were placed in an appropriate environment to allow infection to proceed and then incubated until the disease was ready for assessment. The period between inoculation and assessment varied from four to fourteen days according to the disease and the environment.

Disease control was recorded using the following grading system :

4 = no disease
3 = trace to 5% of disease on untreated plants
2 = 6-25% of disease on untreated plants
1 = 26-59% of disease on untreated plants
0 = 60-100% of disease on untreated plants

The results are shown in Table III.

TABLE III

| COMPOUND NO. | PUCCINIA RECONDITA (WHEAT) | VENTURIA INAEQUALIS (APPLES) | PIRICULARIA ORYZAE (RICE) | CERCOSPORA ARACHIDICOLA (PEANUTS) | PLASMOPARA VITICOLA (VINES) |
|---|---|---|---|---|---|
| 1 | 4 | 2 | 3 | 2 | 4 |
| 4 | 4 | 0 | 4 | 1 | 4 |
| 7 | 0 | 0 | 2 | 0 | 4 |
| 8 | 0 | 0 | 3 | 0 | 3 |
| 9 | 2 | 0 | 2 | 0 | 4 |
| 10 | 0 | 0 | 0 | 2 | 3 |
| 13 | 0 | 2 | 3 | 1 | 3 |
| 14 | 3 | 0 | – | 3 | 4 |
| 15 | 4 | 2 | – | 0 | 4 |
| 16 | 4 | 4 | 3 | 3 | 3 |
| 17 | 4 | 3 | 3 | 2 | 3 |
| 18 | 4 | 0 | 3 | 2 | 4 |
| 19 | 4 | 0 | 4 | 3 | 4 |
| 20 | 3 | 3 | 1 | 2 | 4 |

TABLE III (cont)

| COMPOUND NO. | PUCCINIA RECONDITA (WHEAT) | VENTURIA INAEQUALIS (APPLES) | PIRICULARIA ORYZAE (RICE) | CERCOSPORA ARACHIDICOLA (PEANUTS) | PLASMOPARA VITICOLA (VINES) |
|---|---|---|---|---|---|
| 22 | 3 | 4 | 3 | 3 | 4 |
| 23 | 2 | 0 | 0 | 3 | 4 |
| 24 | 1 | 0 | 0 | 2 | 4 |
| 25 | 3 | 2 | 0 | 0 | 4 |
| 26 | 2 | 0 | 3 | 3 | 4 |
| 27 | 3 | 0 | 0 | 3 | 4 |
| 28 | 4 | 4 | 1 | 3 | 4 |
| 29 | 4 | 0 | 0 | 3 | 4 |
| 31 | 0 | 0 | 2 | 0 | 0 |
| 33 | 0 | 0 | 0 | 0 | 3 |
| 34 | 4 | 0 | 0 | 0 | 4 |
| 35 | 2 | 0 | 0 | 3 | 2 |
| 36 | 3 | - | 0 | - | 4 |
| 37 | 4 | 0 | 0 | 0 | 4 |

TABLE III (cont)

| COMPOUND NO. | PUCCINIA RECONDITA (WHEAT) | VENTURIA INAEQUALIS (APPLES) | PIRICULARIA ORYZAE (RICE) | CERCOSPORA ARACHIDICOLA (PEANUTS) | PLASMOPARA VITICOLA (VINES) |
|---|---|---|---|---|---|
| 38 | 3 | 0 | 0 | 0 | 1 |
| 39 | 4 | 0 | 0 | 3 | 0 |
| 50 | 3 | 1 | 0 | 0 | 4 |
| 51 | 0 | – | 0 | – | 4 |
| 53 | 0 | 0 | 0 | 0 | 3 |
| 54 | 3 | 0 | 0 | 3 | 4 |
| 57 | 3 | 0 | 0 | 0 | 3 |
| 63 | 2 | 0 | 0 | 0 | 4 |
| 64 | 4 | 3 | 0 | 3 | 4 |

"–" means compound not tested

TABLE III (cont)

| COMPOUND NO. | PUCCINIA RECONDITA (WHEAT) | VENTURIA INAEQUALIS (APPLES) | PIRICULARIA ORYZAE (RICE) | CERCOSPORA ARACHIDICOLA (PEANUTS) | PLASMOPARA VITICOLA (VINES) |
|---|---|---|---|---|---|
| 30 | 3 | 0 | 0 | 0 | 1 |
| 39 | 4 | 0 | 0 | 3 | 1 |
| 50 | 3 | 1 | 0 | 0 | 4 |
| 51 | 0 | - | 0 | - | 4 |
| 53 | 0 | 0 | 0 | 0 | 3 |
| 54 | 3 | 0 | 0 | 3 | 4 |
| 55 | 3 | 0 | 0 | 2 | 4 |
| 57 | 2 | 0 | 0 | 0 | 2 |
| 63 | 2 | 0 | 0 | 0 | 4 |
| 64 | 3 | 3 | 0 | 3 | 4 |
| 65 | 4 | 0 | 0 | 3 | 4 |
| 68 | 3 | 0 | 0 | 0 | 4 |
| 73 | 3 | 0 | 0 | 0 | 4 |

TABLE III (cont)

| COMPOUND NO. | PUCCINIA RECONDITA (WHEAT) | VENTURIA INAEQUALIS (APPLES) | PIRICULARIA ORYZAE (RICE) | CERCOSPORA ARACHIDICOLA (PEANUTS) | PLASMOPARA VITICOLA (VINES) |
|---|---|---|---|---|---|
| 76 | 4 | 0 | 0 | 2 | 4 |
| 77 | 3 | 2 | 0 | 2 | 4 |
| 78 | 3 | 0 | 0 | 0 | 4 |
| 81 | 3 | 0 | 0 | 0 | 4 |
| 84 | 2 | 0 | 0 | 0 | 4 |
| 85 | 4 | 0 | 0 | 0 | 4 |
| 86 | 2 | 0 | 1 | 0 | 4 |
| 88 | 0 | 0 | 0 | 2 | 4 |
| 90 | 3 | 0 | 0 | 0 | 4 |
| 91 | 3 | 0 | 0 | 3 | 4 |
| 102 | 3 | 0 | 1 | 3 | 4 |

## EXAMPLE 30

This Example illustrates the activity of the invention compounds against the disease Plasmopara viticola on vines.

For the test on Plasmopara viticola the vine plants (variety ohanez) were grown in John Innes Potting Compost (No. 1 or 2) in 4 cm diameter minipots. The test compounds were formulated either by bead milling with aqueous Dispersol T or as a solution in acetone or acetone/ethanol which was diluted to a concentration of 25 ppm of the compound immediately before use. The solutions or suspensions were applied as a root drench to a final concentration equivalent to approximately 10 ppm compound/dry soil. After 2 days the plants were inoculated with the pathogen, and then placed in a humidity cabinet to allow infection to proceed, and the assessment carried out six days later.

The disease grading system was as for Example 29.

The results are shown in Table IV.

TABLE IV

| COMPOUND NUMBER | PLASMOPARA VITICOLA (VINES) |
|---|---|
| 1 | 4 |
| 4 | 0 |
| 7 | 2* |
| 8 | 1 |
| 9 | 3* |
| 10 | 2 |
| 17 | 0* |
| 18 | 2* |
| 19 | 0* |
| 20 | 4 |
| 22 | 4 |
| 23 | 0 |
| 24 | 0 |
| 25 | 0 |
| 26 | 0 |
| 27 | 4 |
| 28 | 2 |
| 34 | 4 |
| 36 | 4 |
| 37 | 4 |
| 54 | 4 |
| 63 | 4 |
| 85 | 4 |

"*" means the test concentration was 30 ppm

EXAMPLE 31

This Example illustrates the activity of the invention compounds against the disease Phytophthora infestans lycopersicii. Tomato plants (variety Outdoor Girl) were grown in John Innes Potting Compost No. 1 in 4 cm diameter mini pots for 2½-3 weeks. The test compounds were formulated either by bead milling with aqueous Dispersol T or as a solution in acetone/ethanol which was diluted to a concentration of
100 ppm ai. (active ingredient) before use.

After 1 day, the plants were inoculated with the pathogen and placed in a humidity cabinet for 24 hours. They were then transferred to a constant environment chamber to incubate the disease for a further 2 days when the assessment is carried out, using the disease grading system in Example 29. The results are shown in Table V.

TABLE V

| Compound No. | Phytophthora infestans (tomatoes) |
|---|---|
| 20 | 2 |
| 25 | 2 |
| 26 | 3 |
| 33 | 2 |
| 34 | 3 |
| 35 | 3 |
| 36 | 3 |
| 37 | 3 |
| 54 | 3 |
| 57 | 3 |
| 64 | 3 |
| 78 | 2 |
| 102 | 1 |
| 103 | 2 |

1. Compounds having the general formula (I) :

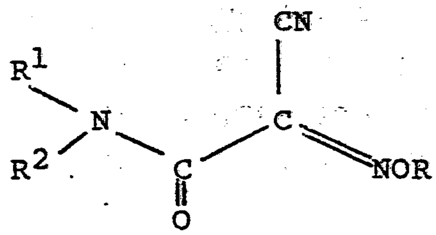

(I)

wherein R is H, alkyl, cycloalkyl or cycloalkylalkyl, optionally substituted alkenyl or optionally substituted alkynyl, optionally substituted benzyl, or a metal ion; $R^1$ is an alkenyl, alkynyl, cyanoalkyl, heterocyclyl or allenylmethyl group, any of which groups can bear substituents or $R^1$ is alkyl substituted by alkoxy, halogen, alkylthio, alkoxycarbonyl, carbamoyl, alkylcarbamoyl or thiocarbamoyl; or $R^1$ is alkyl substituted by thiocarbamoyl and additionally by either optionally substituted aryl or optionally substituted heterocyclyl; $R^2$ is H or is a cyanoalkyl, alkenyl, alkynyl or allenylmethyl group, any of which groups can bear substituents.

2. Compounds having the general formula (I) :

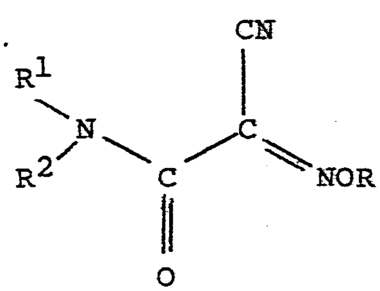

(I)

wherein R is H, alkyl, alkenyl, or alkynyl; $R^1$ is alkenyl, alkynyl, alkoxyalkyl, haloalkyl, alkylthioalkyl, cyanoalkyl, alkoxycarbonylalkyl,

alkylcarbamoylalkyl, heterocyclyl, benzyl or allenylmethyl; and $R^2$ is H, alkenyl, alkynyl or allenylmethyl.

3. Compounds having the general formula (I) :

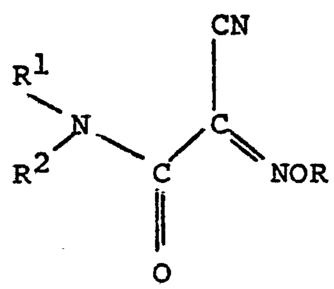

(I)

wherein R is H, alkyl, cycloalkyl or cycloalkylalkyl, optionally substituted alkenyl or alkynyl, optionally substituted benzyl, or a metal ion; $R^1$ is an optionally substituted alkenyl, alkynyl, cyanoalkyl, heterocyclyl or allenylmethyl group or is alkyl substituted by alkoxy, halogen, alkylthio, alkoxycarbonyl, carbamoyl, alkylcarbamoyl or thiocarbamoyl; $R^2$ is H or is an optionally substituted cyanoalkyl, alkenyl, alkynyl or allenylmethyl group.

4. Compounds as claimed in claim 1 having the general formula :

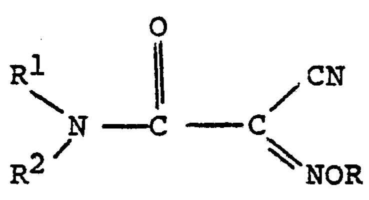

(I)

wherein R is hydrogen, $C_{1-12}$ alkyl, $C_{3-6}$ cycloalkyl, ($C_{3-6}$ cycloalkyl) $C_{1-4}$ alkyl, $C_{3-5}$ alkenyl or $C_{3-5}$ alkynyl either of which may be optionally substituted with one or more $C_{1-4}$ alkyl groups or halogens, or R is benzyl optionally substituted with one or more

halogen, $C_{1-4}$ alkyl, $C_{1-4}$ alkoxy, halo $C_{1-4}$ alkyl, or nitro groups, or R is a metal ion; $R^1$ is $C_{3-10}$ alkenyl or $C_{3-10}$ alkynyl either of which may be substituted with one or more $C_{1-4}$ alkyl, halogen, or $C_{1-4}$ alkoxy groups, or $R^1$ is $C_{1-10}$ alkyl optionally substituted with one or more $C_{1-4}$ alkoxy, cyclic alkoxy (one or more oxygen atoms in a 5-or 6-membered ring), halogen, $C_{1-4}$ alkylthio, $C_{1-5}$ alkoxy carbonyl, carbamoyl, $C_{1-5}$ alkylcarbamoyl, or thiocarbamoyl groups; or $R^1$ is cyano $C_{1-10}$ alkyl, the alkyl moiety of which is optionally substituted with $C_{3-5}$ alkenyloxy, $C_{3-5}$ alkynyloxy or $C_{1-4}$ alkoxy, all of these three substituents being themselves optionally substituted with $C_{1-4}$ alkyl, halo $C_{1-4}$ alkyl, halogen or cyano groups, or $R^1$ is cyano $C_{1-10}$ alkyl optionally substituted by aryl, the aryl group being substituted with $C_{1-4}$ alkyl, halogen, $C_{1-4}$ alkoxy, nitro, cyano or halo $C_{1-4}$ alkyl, or $R^1$ is a cyano $C_{1-10}$ alkyl group optionally substituted by a 5- or 6-membered heterocyclyl group which may be linked through a carbon or nitrogen atom in the ring and which ring may be optionally substituted with the substituents recited for aryl, or $R^1$ is $C_{4-10}$ allenylmethyl optionally substituted with one or more $C_{1-4}$ alkyl groups or halogen atoms; or $R^1$ is $C_{1-10}$ alkyl substituted by thiocarbamoyl and, additionally, substituted by either aryl, the aryl group itself being optionally substituted with $C_{1-4}$ alkyl, halogen, $C_{1-4}$ alkoxy, nitro, halo $C_{1-4}$ alkyl, or cyano, or additionally substituted by heterocyclyl, the heterocyclyl group being optionally substituted by $C_{1-4}$ alkyl, halogen, nitro, halo $C_{1-4}$ alkyl or cyano; and $R^2$ is hydrogen or a cyanoalkyl, alkenyl, alkynyl or

allenylmethyl group as defined above for $R^1$ and optionally substituted as defined above for $R^1$.

5. Compounds as claimed in claim 1 or claim 4 wherein R is hydrogen, methyl, ethyl, propyl, butyl, allyl, propargyl, haloallyl, halopropargyl, benzyl, halobenzyl, (halo $C_{1-4}$ alkyl) benzyl, ($C_{1-4}$ alkoxy)benzyl, nitrobenzyl, $C_{3-6}$ cycloalkyl, ($C_{3-6}$ cycloalkyl) $C_{1-4}$ alkyl, or a metal ion;

$R^1$ is propargyl, halopropargyl, allyl, haloallyl, allenylmethyl, $C_{4-5}$ alkynyl, ($C_{1-4}$ alkoxy) $C_{1-4}$ alkyl, cyano $C_{1-10}$ alkyl, cyano $C_{3-6}$ cycloalkyl, $C_{1-4}$ alkoxycarbonyl $C_{1-4}$ alkyl, carbamoyl $C_{1-4}$ alkyl, thiocarbamoyl $C_{1-4}$ alkyl, heterocyclyl, heterocyclyl $C_{1-4}$ alkyl, heterocyclyl (cyano) $C_{1-4}$ alkyl, heterocyclyl (thiocarbamoyl) $C_{1-4}$ alkyl, halophenyl (cyano) $C_{1-4}$ alkyl, cyano (carbamoyl) $C_{1-4}$ alkyl, cyano (thiocarbamoyl) $C_{1-4}$ alkyl, cyano ($C_{1-4}$ alkoxy carbonyl) $C_{1-4}$ alkyl, cyano ($C_{1-4}$ alkoxy) $C_{1-4}$ alkyl, $C_{1-4}$ alkylthio $C_{1-4}$ alkyl, halo $C_{1-4}$ alkyl, cyano ($C_{3-4}$ alkynyloxy)$C_{1-4}$ alkyl, cyano ($C_{3-4}$ alkenyloxy) $C_{1-4}$ alkyl; and $R^2$ is hydrogen, $C_{1-4}$ alkyl, allyl, haloallyl, propargyl, halopropargyl, or cyano $C_{1-4}$ alkyl.

6. Compounds as claimed in any of claims 1 to 5 wherein the heterocyclyl function or moiety is :

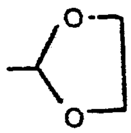 , 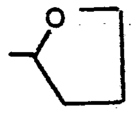 , 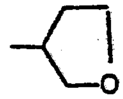 ,

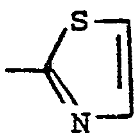 , 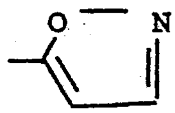 , 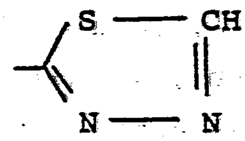 ,

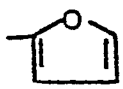 , 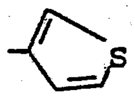 , 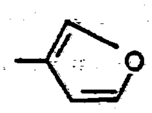 ,

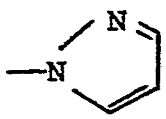 , 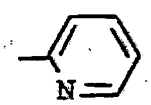 , 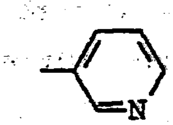

or 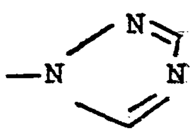

and is optionally substituted with a $C_{1-4}$ alkyl group.

7. Compounds as claimed in any of claims 1 to 6 wherein R is $C_{1-4}$ alkyl, allyl or propargyl; $R^2$ is hydrogen; and $R^1$ is cyano $C_{1-4}$ alkyl, thiocarbamoyl $C_{1-4}$ alkyl, cyano (furyl) $C_{1-4}$ alkyl or cyano (thienyl) $C_{1-4}$ alkyl.

8. Compounds as claimed in claim 7 wherein R is methyl, ethyl or propargyl; $R^2$ is hydrogen; and $R^1$ is cyano $C_{1-2}$ alkyl, thiocarbamoyl methyl, cyano (2-furyl) methyl, cyano (3-furyl) methyl, cyano (3-thienyl)methyl.

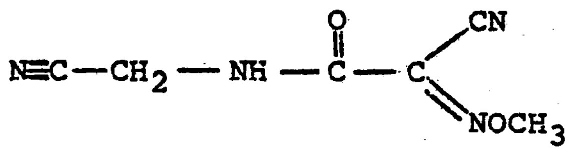

(Compound No. 20 of Table I)

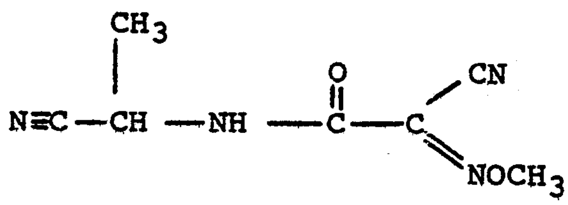

(Compound No. 34 of Table I)

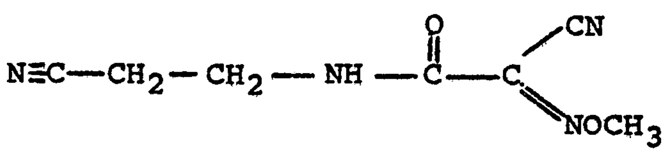

(Compound No. 36 of Table I)

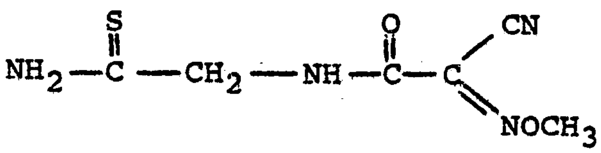

(Compound No. 54 of Table I)

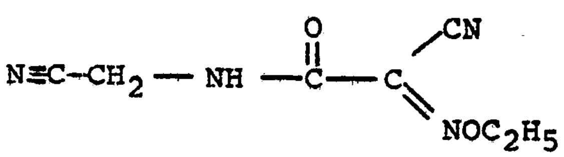

(Compound No. 22 of Table I)

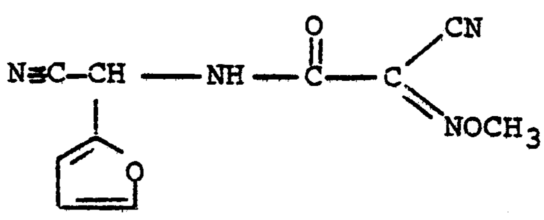

(Compound No. 63 of Table I)

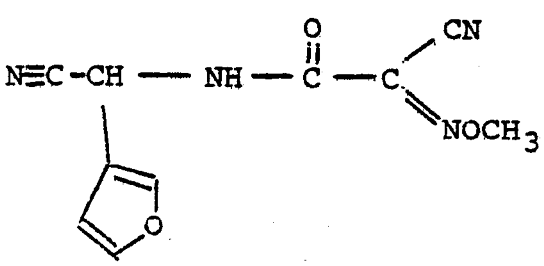

(Compound No. 85 of Table I)

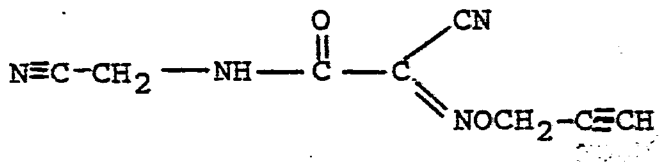

(Compound No. 27 of Table I)

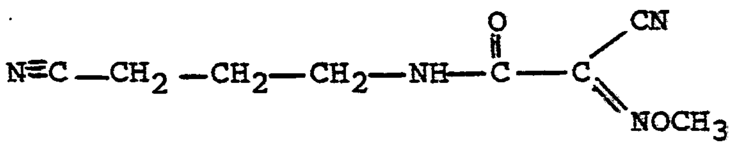

(Compound No. 37 of Table I)

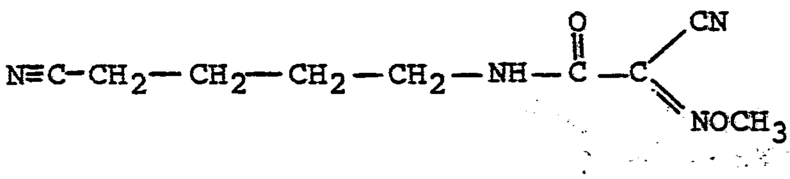

(Compound No. 68 of Table I)

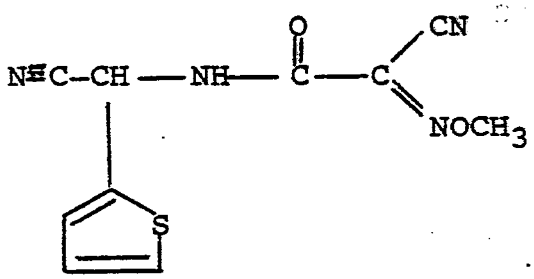

(Compound No. 84 of Table I)

10. A process for preparing compounds as claimed in any of claims 1 to 9 which comprises

(a) reacting an ester of formula (III)

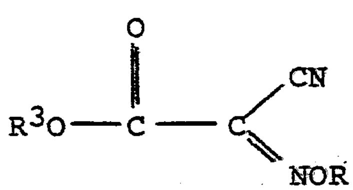

(III)

wherein R is as defined in any of claims 1 to 8 and $R_3$ is $C_{1-10}$ alkyl with an amine $R^1R^2NH$; wherein $R^1$ and $R^2$ are as defined in any of claims 1 to 8, or

(b) reacting an acid chloride of formula V

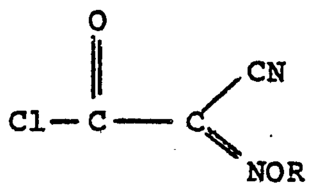

(V)

wherein R is as defined in any of claims 1 to 8, with an amine $R^1R^2NH$; wherein $R^1$ and $R^2$ are as defined in any of claims 1 to 8, or

(c) reacting compounds of formula VI

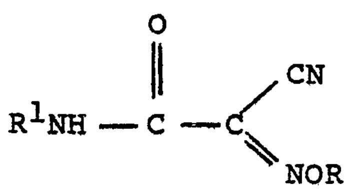

(VI)

wherein R and $R^1$ are as defined in any of claims 1 to 8, with a strong base in the presence of an alkyl halide $R^2X$; wherein $R^2$ is as defined in any of claims 1 to 8 and X is a halogen, or

(d) reacting an ester of formula III

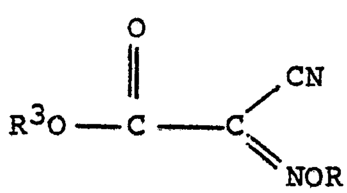

(III)

wherein R is as defined in any of claims 1 to 8 and $R^3$ is $C_{1-10}$ alkyl, with an amine anion of formula IX

$$R^4NH^-M^+ \qquad (IX)$$

where $R^4$ is a heterocyclyl radical and $M^+$ is an alkali metal anion, generated by the action of a strong base on an amine $R^4NH_2$, or

(e) reacting an oxime of formula XI

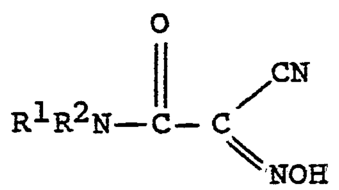

(XI)

with a base and thereafter with a compound RX where R is as defined in any of claims 1 to 8 and X is a halogen or a sulphonate, or RX is a dialkylsulphate, or

(f) reacting a compound of formula I

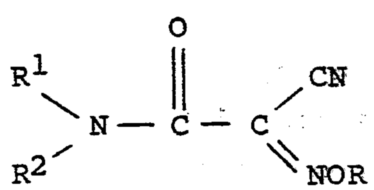

(I)

where R is as defined in any of claims 1 to 8 and $R^1$ is alkenyl or alkynyl and $R^2$ is hydrogen, alkenyl or alkynyl with a halogen, $X_2$, to produce compounds of formula I wherein $R^1$ and/or $R^2$ are dihaloalkyl or dihaloalkenyl, or

(g) reacting compounds of formula I

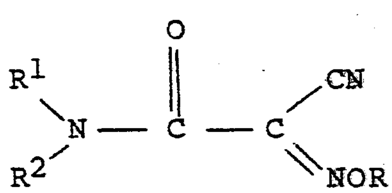

(I)

where R and $R^2$ are as defined in any of claims 1 to 8 and $R^1$ is a cyanoalkyl group, with hydrogen sulphide gas in the presence of an amine and, if necessary, a solvent to produce compounds of formula I wherein $R^1$ is an alkyl group substituted with thiocarbamoyl, or

(h) reacting an amine $R^1R^2NH$, where $R^1$ and $R^2$ are as defined in any of claims 1 to 8 (but are not thiocarbamoylalkyl), with an acid chloride $R^6OCOCl$ where $R^6$ is an optionally substituted benzyl group or is $C_{1-4}$ alkyl, in either a single or two phase system and in the presence of an acid acceptor and a solvent or solvents, to produce an amide of formula XV

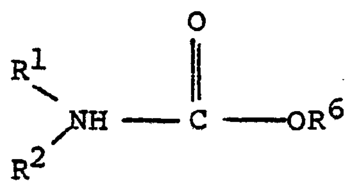

(XV)

wherein $R^1$ is a carbamoylalkyl group, which is then subjected to a thionation reaction to convert $R^1$ to a thiocarbamoylalkyl group, the resultant thioamide being then reacted with a hydrogen halide, HX, where X is chlorine or bromine to produce the hydrogen halide salt

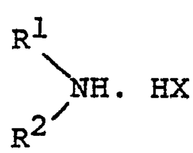

(XVII)

which is then reacted with an acid chloride of formula V

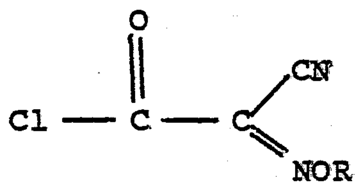

in a single or two phase system in the presence of a solvent, or solvents, and an acid acceptor to produce compounds of formula I wherein $R^1$ is an alkyl group substituted with thiocarbamoyl and $R^2$ and R are as defined in any of claims 1 to 8, or

(i) brominating or chlorinating a compound of formula XXI

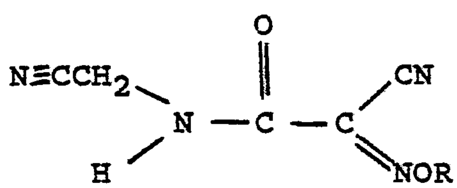

(XXI)

to produce a compound of formula XXII

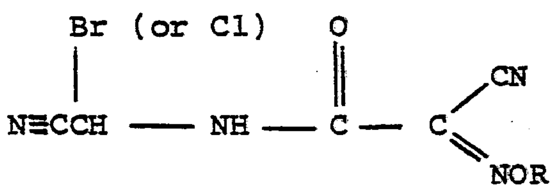

(XXII)

which is then reacted with a compound $R^5H$ where $R^5$ is a heterocyclic ring containing from 2 to 4 nitrogen atoms or is an alkoxy, alkenyloxy or alkynyloxy group as defined in any of claims 1 to 8, followed by an acid acceptor, or

(j) reacting a compound of formula XXIII

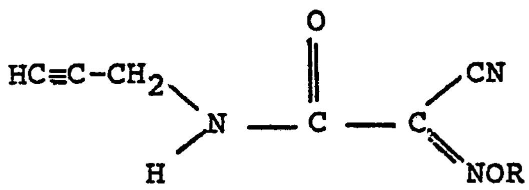

(XXIII)

wherein R is as defined in any of claims 1 to 8 with formaldehyde, a copper $^{I}$ salt and a secondary amine in the presence of a solvent and, if necessary, under an inert atmosphere, to produce compounds of formula XXIV:

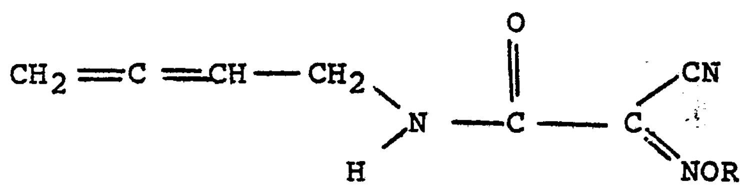

(XXIV)

or

(k) reacting a compound of formula XXV

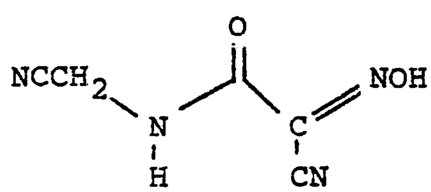

(XXV)

with aqueous sodium hydroxide, followed by a metal halide $MX_m$ where M is a metal, X a halogen and m is an integer of one to three to produce compounds of formula XXVI :

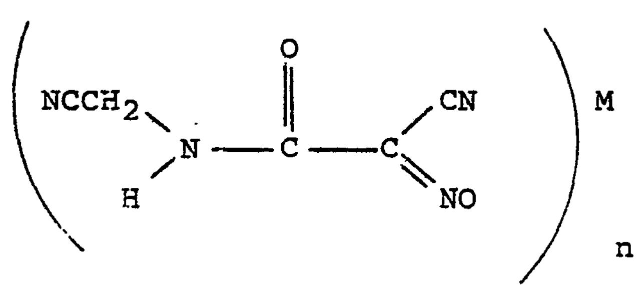

where n is an integer of one to three.

11. A fungicidal composition comprising as an active ingredient a compound as claimed in any of claims 1 to 9.

12. A process for combating fungi which comprises applying a plant or to seeds thereof, or to the locus of the plant or seeds, a compound as claimed in any of claims 1 to 9 or a composition as claimed in claim 11.

European Patent Office

# EUROPEAN SEARCH REPORT

0201999
Application number

EP 86301986.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | AT - B - 370 091 (CIBA-GEIGY)<br>* Claim 1 *<br>-- | 1,10 | C 07 C 131/00<br>C 07 C 153/057<br>C 07 D 333/20<br>C 07 D 333/24<br>C 07 D 261/14<br>C 07 D 249/08<br>C 07 D 317/32<br>C 07 D 277/46<br>C 07 D 285/12<br>C 07 D 231/12<br>C 07 D 307/04<br>C 07 D 307/54<br>C 07 D 213/57<br>A 01 N 37/50<br>A 01 N 43/00 |
| A | EP - A1 - 0 072 970 (BAYER)<br>* Claims 1,2,7,8 *<br>-- | 1,10-12 | |
| A | DE - A1 - 2 626 828 (BAYER)<br>* Claims 1-4 *<br>-- | 1,10-12 | |
| A | US - A - 3 919 284 (KANG LIN)<br>* Abstract; column 1, lines 5-9 *<br>-- | 1 | |
| A | DE - A1 - 3 322 010 (BASF)<br>* Claims 1,3,4,6 *<br>-- | 1,10-12 | **TECHNICAL FIELDS SEARCHED (Int. Cl.4)** |
| A | EP - A2 - 0 129 170 (BASF)<br>* Abstract *<br>-- | 1 | C 07 C 131/00<br>C 07 C 153/00<br>C 07 D 333/00<br>C 07 D 261/00<br>C 07 D 249/00<br>C 07 D 317/00<br>C 07 D 277/00<br>C 07 D 285/00<br>C 07 D 231/00<br>C 07 D 307/00<br>C 07 D 213/00 |
| A | DD - A - 149 523 (AKADEMIE DER WISSENSCHAFTEN)<br>* Abstract; claim 1 *<br>-- | 1,6, 10-12 | |
| A | GB - A - 1 390 840 (ABBOTT LABORATORIES)<br>* Claims 1,4 *<br>-- | 1,6, 10 | |

The present search report has been drawn up for all claims

| Place of search | Date of completion of the search | Examiner |
|---|---|---|
| VIENNA | 26-06-1986 | REIF |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

European Patent Office

# EUROPEAN SEARCH REPORT

0201999
Application number

-2-
EP 86301986.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US - A - 4 281 134 (PIETER TEN HAKEN et al.)<br>* Claim 1; column 2, line 25 *<br>-- | 1,6,10-12 | |
| A | EP - A2 - 0 104 691 (SHELL INTERNATIONALE)<br>* claims 1,13,15 *<br>---- | 1,6,10-12 | |
| | | | TECHNICAL FIELDS SEARCHED (Int. Cl.4) |

The present search report has been drawn up for all claims

| Place of search | Date of completion of the search | Examiner |
|---|---|---|
| VIENNA | 26-06-1986 | REIF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82